# EUROPEAN PATENT SPECIFICATION

(11) **EP 4 146 160 B1**
(45) Date of publication and mention of the grant of the patent: **01.01.2025**
(21) Application number: 21724283.3
(22) Date of filing: 07.05.2021
(51) Int. Cl.: A61K 8/73, A61Q 11/00, A61Q 19/10, A61K 8/02

(54) **COSMETIC COMPOSITION COMPRISING CELLODEXTRINS**
KOSMETISCHE ZUSAMMENSETZUNG MIT CELLODEXTRINEN
COMPOSITION COSMÉTIQUE COMPRENANT DES CELLODEXTRINES

(30) Priority: 08.05.2020 EP 20173630
(43) Date of publication of application: 15.03.2023
(73) Proprietor: Pfeifer & Langen GmbH & Co. KG, 50858 Köln (DE)
(72) Inventor: HÄSSLER, Thomas, 50997 Köln (DE)
(74) Representative: Kutzenberger Wolff & Partner
(86) International application number: PCT/EP2021/062167
(87) International publication number: WO 2021/224463

(56) References cited:
- WO-A1-2008/023145
- WO-A1-2016/094246

## Description

Priority is claimed of European patent application no. 20 173 630.3, filed on May 8, 2020.

### FIELD OF THE INVENTION

The invention relates to a cosmetic composition comprising a cellodextrin component comprising a cellosaccharide selected from the group consisting of cellobiose, cellotriose, cellotetraose, cellopentaose, cellohexaose, celloheptaose, cellooctaose, cellononaose, cellodecaose, celloundecaose, cellododecaose and combinations thereof; and a surfactant component comprising an anionic surfactant, cationic surfactant, amphoteric surfactant, nonionic surfactant, or any combination thereof; wherein at least a portion of said cellodextrin component is present within the cosmetic composition in undissolved form, wherein the cosmetic composition has a turbidity of at least 5 NTU, determined by nephelometric analysis.

### BACKGROUND ART

In many cosmetic compositions, especially in soaps, wash lotions, peeling or scrub products, and tooth pastes raw materials such as polyethylene, polyurethane, polyamide, polyacrylates, and others are used, typically for functions such as exfoliating, cleansing, viscosity modification, optical appearance modification (e.g. white creamy optical effect) and others. Typically, these raw materials are present in form of small particles, also referred to as microplastic.

Such microplastic-containing cosmetic compositions have two major drawbacks: They are usually based on non-renewable resources and, especially when used in rinse-off cosmetics such as peelings, scrubs, tooth pastes or masks, are harmful for the environment. Environmental-friendly alternatives such as shells & kernels, fruits, seeds, minerals and others are known.

Cello dextrin (cellosaccharide)-containing compositions are known from the prior art. EP 1 930 012 relates to a cellooligosaccharide-containing composition for prevention or improvement of lifestyle-related diseases in the fields of foods and internal medicines.

However, the known alternatives are not satisfactory in every respect, especially when it comes to consumer acceptance. Many cosmetic compositions cannot be rinsed off easily and quickly, especially if they have a high content of hydrophobic ingredients such as lipoids. There is therefore a demand to improve washability of such cosmetic formulations. While washability can be improved by adding strong surfactants, such strong surfactants may have a detrimental effect on the skin, as they dissolve any oily substances, i.e. also those lipoids that are beneficial to the skin and naturally present in healthy skin. Further, many cosmetic compositions after use leave an unpleasant feel of the skin. For example, skin scrub creams (peelings) are used for mechanical exfoliation, i.e. removal of the oldest dead skin cells on the skin's outermost surface by abrasion. Several of these cosmetic compositions after use leave an unpleasant feel of the skin and thus suffer from poor consumer acceptance.

There is a demand for cosmetic compositions having a low content of microplastic or do not contain microplastic at all and which have improved consumer acceptance, i.e. with regard to quick and easy washability and the effect on skin, compared to the cosmetic compositions known from the prior art.

Toothpastes generally include an abrasive material which is dispersed in a gel or paste base. Abrasives remove stains and plaque, as well as polish teeth. Common abrasives include calcium phosphates, alumina, calcium carbonate, and silica. Toothpaste must be abrasive enough to remove plaque and stains, but should not be so abrasive as to damage tooth enamel. Toothpastes are generally classified into two types: Opaque and transparent. Opaque toothpastes are usually white or colored. Such toothpastes usually contain calcium-based abrasives, in particular chalk. Gel toothpastes usually contain abrasive silica. Also known are multiphase toothpastes, which have an opaque phase and a transparent phase. Toothpastes, in particular-chalk based types, also contain thickening silica. Silica helps build up the viscosity. However, the thickening silica-based agent, especially at higher levels, may adversely affect the flavor delivery. This often compels formulators to dose an appropriate overage of the flavor. Since flavors are very expensive, even a slight reduction in flavor can be economically significant. Therefore, it is desirable to either reduce the level of thickening silica, or preferably, have no thickening silica. Toothpastes usually contain some polymers that are used for the delivery of flavor. One of the disadvantages of such polymers is that the unbalanced amount of such polymers can affect the spreadability of the paste. Furthermore, the paste becomes unstable after about a month of storage at elevated temperature. An arbitrary reduction in the level of such polymers cannot be a viable solution, as it may adversely affect delivery of flavor.

There is a demand for cosmetic compositions such as tooth pastes having optimized optical appearance, abrasive capacity, spreadability, storage stability and flavor delivery.

It is an object of the invention to provide cosmetic compositions having advantages compared to the cosmetic compositions of the prior art. The cosmetic compositions should be friendly to the environment and have improved consumer acceptance.

This object has been achieved by the subject-matter of the patent claims.

### SUMMARY OF THE INVENTION

The invention relates to a cosmetic composition comprising a cellodextrin component comprising a cellosaccharide selected from the group consisting of cellobiose, cellotriose, cellotetraose, cellopentaose, cellohexaose, celloheptaose, cellooctaose, cellononaose, cellodecaose, celloundecaose, cellododecaose and combinations thereof; and
a surfactant component comprising an anionic surfactant, cationic surfactant, amphoteric surfactant, nonionic surfactant, or any combination thereof;
wherein at least a portion of said cellodextrin component is present within the cosmetic composition in undissolved form,
wherein the cosmetic composition has a turbidity of at least 5 NTU, determined by nephelometric analysis.

The cosmetic compositions according to the invention are useful as skin and tooth cleansing compositions to be rinsed off with water after topical application to the skin and cleaning. Further, the cosmetic compositions according to the invention are useful as skin care composition to remain on the skin after topical application, e.g. exfoliating scrub soaps, body scrubs, peels (face peels, hand peels, foot peels), handwashing pastes, liquid soaps, shower gels, hair-removal compositions, face masks, and antiacne compositions and compositions suitable for keratin materials made from micronized biodegradable polymers and body lotions and cremes or powders and make-up. The compositions according to the invention are intended more particularly for application to human keratin materials, such as the skin (including the scalp), the nails and keratin fibers, and/or mucous membranes.

It has been surprisingly found that cosmetic compositions can be prepared containing a cellodextrin component, wherein at least a portion of said cellodextrin component is present within the cosmetic composition in undissolved form. It has been surprisingly found that such cosmetic compositions have excellent scrubbing properties, especially when the cellodextrin component that is present within the cosmetic composition in undissolved form forms particles of a suitable size to act as abrasive substance in mechanical exfoliation.

Further, it has been surprisingly found that the cosmetic compositions according to the invention have excellent consumer acceptance when compared with other cosmetic compositions of the same type not containing cellodextrin component. Still further, it has been surprisingly found that the cosmetic compositions according to the invention provide easy and also quick washability. Yet further, it has been surprisingly found that the cosmetic compositions according to the invention provide excellent skin effect/skin feel.

Furthermore, it has been surprisingly found that the cellodextrin component that is present within the cosmetic composition in undissolved form can provide the cosmetic composition with a white, creamy optical appearance, especially when the cellodextrin component is present in finely dispersed form. As cellodextrin is biologically degradable, the present invention makes it possible to avoid synthetic polymers that otherwise would be harmful for the environment as they would enter the environment in form of microplastic.

### DETAILED DESCRIPTION OF THE INVENTION

A first aspect of the invention relates to a cosmetic composition comprising a cellodextrin component comprising a cellosaccharide selected from the group consisting of cellobiose, cellotriose, cellotetraose, cellopentaose, cellohexaose, and combinations thereof;
a surfactant component comprising an anionic surfactant, cationic surfactant, amphoteric surfactant, nonionic surfactant, or any combination thereof;
wherein at least a portion of said cellodextrin component is present within the cosmetic composition in undissolved form, wherein the cosmetic composition has a turbidity of at least 5 NTU, determined by nephelometric analysis.

Unless expressly stated otherwise, all percentages are weight percent. For the purpose of the specification, unless expressly stated otherwise, "essentially consisting of" preferably refers to a content of at least 95 wt.-%, more preferably at least 98 wt.-%, still more preferably at least 99.0 wt.-%. Unless expressly stated otherwise, all norms such as EN ISO, DIN or ASTM referred to herein, are referred to in the version that is valid on January 1, 2021.

For the purpose of the specification, cosmetic compositions encompass any composition that is intended to be applied to the human body for cleansing, beautifying, promoting attractiveness, or altering the appearance without affecting the body's structure or functions.

Cosmetic compositions according to the invention include various forms and product types. Cosmetic compositions according to the invention include solutions and dispersions, whereas the dispersions may be suspensions or emulsions, whereas the emulsions may be water-in-oil emulsions or oil-in-water emulsions and powdery forms. Any combinations are also contemplated according to the invention.

Non limiting examples of cosmetic compositions according to the invention include solutions (e.g. shampoos, body wash, hand cleansers such as foam soap and liquid soap, mascaras, eye liners, colognes), creams/emulsions (e.g. hand moisturizers, make up, hair conditioners, sunscreens), lotions (e.g. facial moisturizers, leave-in hair conditioners and moisturizing cleansers), suspensions (e.g. sunscreens, hand washes and shampoos), ointments/pastes, powders (e.g. aby powder, eye powder, foot powder), gels (hair products, body washes, shaving products and toothpastes), sticks, tablets, cakes, capsules (e.g. compact powder, eye shadow, cheek shadow cakes), exfoliating products (e.g. exfoliating scrub soap, body scrub, peel such as face peel, hand peel, foot peel, handwashing paste, tooth paste, hair-removal composition, face mask, or antiacne compositions).

The cosmetic composition according to the invention comprises a cellodextrin component comprising a cellosaccharide selected from the group consisting of cellobiose, cellotriose, cellotetraose, cellopentaose, cellohexaose, celloheptaose, cellooctaose, cellononaose, cellodecaose, celloundecaose, cellododecaose, and combinations thereof. Cellodextrins (sometimes also referred to as "cellosaccharides") are known to the skilled person. Cellodextrins are classified by the degree of polymerization (DP) which indicates the number of linked glucose monomers it contains, wherein each glucose monomer is linked via a beta-1,4 glycosidic bond; cellobiose (DP=2), cellotriose (DP=3), cellotetraose (DP=4), cellopentaose (DP=5), cellohexaose (DP=6), celloheptaose (DP=7), cellooctaose (DP=8), cellononaose (DP=9), cellodecaose (DP=10), celloundecaose (DP=11), cellododecaose (DP=12).

In preferred embodiments of the cosmetic composition according to the invention, the total content of the cellodextrin component is at least 0.01 wt.-%, or at least 0.1 wt.-%, or at least 1.0 wt.-%, preferably at least 5.0 wt.-%, more preferably at least 10 wt.-%, still more preferably at least 15 wt.-%, yet more preferably at least 20 wt.-%, even more preferably at least 25 wt.-%, yet more preferably at least 30 wt.-%, and most preferably at least 35 wt.-%, in each case relative to the total weight of the cosmetic composition.

In preferred embodiments of the cosmetic composition according to the invention, the weight content of the cellodextrin component is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In a preferred embodiment, the cellodextrin component comprises or essentially consists of cellobiose (D=2).

In preferred embodiments of the cosmetic composition according to the invention, the weight content of cellobiose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellobiose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellobiose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellobiose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellotriose (DP=3).

In preferred embodiments of the cosmetic composition according to the invention, the weight content of cellotriose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellotriose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellotriose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellotriose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellotetraose (DP=4).

In preferred embodiments of the cosmetic composition according to the invention, the weight content of cellotetraose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellotetraose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellotetraose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellotetraose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellopentaose (DP=5).

In preferred embodiments of the cosmetic composition according to the invention, the weight content of cellopentaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellopentaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellopentaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellopentaose is at most 90 wt. -%, or at most 80 wt. -%, at most 70 wt. -%, or at most 60 wt. -%, at most 50 wt. -%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellohexaose (DP=6).

In preferred embodiments of the cosmetic composition according to the invention, the weight content of cellohexaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellohexaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellohexaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellohexaose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of celloheptaose (DP=7).

In preferred embodiments of the cosmetic composition according to the invention, the weight content of celloheptaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of celloheptaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of celloheptaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of celloheptaose is at most 90 wt. -%, or at most 80 wt. -%, at most 70 wt. -%, or at most 60 wt. -%, at most 50 wt. -%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellooctaose (DP=8).

In preferred embodiments of the cosmetic composition according to the invention, the weight content of cellooctaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellooctaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellooctaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellooctaose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellononaose (DP=9).

In preferred embodiments of the cosmetic composition according to the invention, the weight content of cellononaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellononaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellononaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellononaose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellodecaose (DP=10).

In preferred embodiments of the cosmetic composition according to the invention, the weight content of cellodecaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellodecaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellodecaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellodecaose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of celloundecaose (DP=11).

In preferred embodiments of the cosmetic composition according to the invention, the weight content of celloundecaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of celloundecaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of celloundecaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of celloundecaose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In a preferred embodiment, the cellodextrin component comprises or essentially consists of cellododecaose (DP=12).

In preferred embodiments of the cosmetic composition according to the invention, the weight content of cellododecaose is at least at least 0.01 wt.-%, or at least 0.05 wt.-%, or at least 0.1 wt.-%, or at least 0.5 wt.-%, or at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

In preferred embodiments of the cosmetic composition according to the invention, the weight content of cellododecaose is at most 45 wt.-%, or at most 42.5 wt.-%, or at most 40 wt.-%, or at most 37.5 wt.-%, or at most 35 wt.-%, or at most 32.5 wt.-%, or at most 30 wt.-%, or at most 27.5 wt.-%, or at most 25 wt.-%, or at most 22.5 wt.-%, in each case relative to the total weight of the cosmetic composition.

In preferred embodiments of the cosmetic composition according to the invention, the weight content of cellododecaose is at least 10 wt.-%, or at least 20 wt.-%, at least 30 wt.-%, or at least 40 wt.-%, at least 50 wt.-%, or at least 60 wt.-%, at least 70 wt.-%, or at least 80 wt.-%, at least 90 wt.-%, or at least 95 wt.-%, in each case relative to the total weight of the cellodextrin component.

In preferred embodiments of the cosmetic composition according to the invention, the weight content of cellododecaose is at most 90 wt.-%, or at most 80 wt.-%, at most 70 wt.-%, or at most 60 wt.-%, at most 50 wt.-%, or at most 40 wt.-%, at most 30 wt.-%, or at most 20 wt.-%, at most 10 wt.-%, or at most 5.0 wt.-%, in each case relative to the total weight of the cellodextrin component.

In preferred embodiments, the cosmetic composition according to the invention essentially comprises neither cellotriose, nor cellotetraose, nor cellopentaose, nor cellohexaose, nor celloheptaose, nor cellooctaose, nor cellononaose, nor cellodecaose, nor celloundecaose, nor cellododecaose.

At least a portion of the cellodextrin component which is present in the cosmetic composition according to the invention is present in undissolved form.

Suitable methods for determining undissolved constituents in liquid compositions are known to the skilled person. For example, the presence of an undissolved portion of the cellodextrin component in the cosmetic composition according to the invention may be detectable through the naked eye or by means of turbidity measurements or absorbance-based optical density measurements using a nephelometer, spectrophotometer or Near-Infrared (NIR) spectrometer, e.g. using a Dencytee^{®} Sensor by the Hamilton Company (e.g. Dencytee^{®} Unit DN12 - 120 having a length of 120 mm).

In preferred embodiments of the cosmetic composition according to the invention, the portion of the cellodextrin component that is present in undissolved form amounts to at least 5.0 wt.-%, or at least 10 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.%, in each case of the total amount of the cellodextrin component that is contained in the cosmetic composition.

In preferred embodiments of the cosmetic composition according to the invention, the portion of the cellodextrin component that is present in dissolved form amounts to at least 5.0 wt.-%, or at least 10 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, in each case of the total amount of the cellodextrin component that is contained in the cosmetic composition.

Methods for the quantification of the amount of the cellodextrin component that is present in undissolved form are also known to the skilled person, e.g. high-pressure liquid chromatography coupled with refractive index detector (HPLC-RID), gas chromatography mass spectrometry (GC/MS) or quantitative proton (¹H) nuclear magnetic resonance spectroscopy.

Preferably, the cosmetic composition comprises a multitude of solid particles, wherein at least a portion of the multitude of solid particles comprises at least a portion of the cellodextrin component (in the following said at least portion of solid particles is also referred to as "cellodextrin-containing particles").

Preferably, the solid particles which comprise at least a portion of the cellodextrin component (i.e. the cellodextrin-containing particles) essentially consist of the cellodextrin component.

Preferably, substantially all particles of the multitude of solid particles comprise at least a portion of the cellodextrin component, i.e. preferably all particles contained in the cosmetic composition according to the invention are cellodextrin-containing particles.

Preferably, substantially the total amount of the cellodextrin component which is comprised in the cosmetic composition is comprised in the multitude of solid particles (i.e. in the cellodextrin-containing particles).

In preferred embodiments of the cosmetic composition according to the invention, the particles within the multitude of solid particles, i.e. the cellodextrin-containing particles, have a D50(v) value of at least 0.001 µm, or at least 0.005 µm, or at least 0.01 µm, or at least 0.05 µm, or at least 0.1 µm, or at least 0.5 µm, or at least 1.0 µm, or at least 5.0 µm, or at least 10 µm, or at least 50 µm, or at least 100 µm, determined by laser diffraction analysis according to ISO 13 320.

In preferred embodiments of the cosmetic composition according to the invention, the particles within the multitude of solid particles, i.e. the cellodextrin-containing particles, have a D50(v) value of at most 100 µm, or at most 50 µm, or at most 10 µm, or at most 5.0 µm, or at most 1.0 µm, or at most 0.5 µm, or at most 0.1 µm, or at most 0.05 µm, or at most 0.01 µm, or at most 0.005 µm, or at most 0.001 µm, determined by laser diffraction analysis according to ISO 13 320.

In preferred embodiments of the cosmetic composition according to the invention, the particles within the multitude of solid particles, i.e. the cellodextrin-containing particles, have an
- D 10(v) value within the range of from 0.1 µm to 2500 µm; preferably from 1000 µm to 2000 µm, or from 500 µm to 1000 µm, or from 200 µm to 500 µm, or from 75 µm to 200 µm, or from 0.1 µm to 4 µm, or from 0.3 µm to 1.0 µm, or from 0.3 µm to 0.8 µm; and/or
- D50(v) value within the range of from 0.1 µm to 2500 µm; preferably from 1000 µm to 2000 µm, or from 500 µm to 1000 µm, or from 200 µm to 500 µm, or from 75 µm to 200 µm, or from 0.1 µm to 4 µm, or from 0.3 µm to 1.0 µm, or from 0.3 µm to 0.8 µm; and/or
- D90(v) value within the range of from 0.1 µm to 2500 µm; preferably from 1000 µm to 2000 µm, or from 500 µm to 1000 µm, or from 200 µm to 500 µm, or from 75 µm to 200 µm, or from 0.1 µm to 4 µm, or from 0.3 µm to 1.0 µm, or from 0.3 µm to 0.8 µm;
determined by laser diffraction analysis according to ISO 13 320.

Preferably, the particles within the multitude of solid particles, i.e. the cellodextrin-containing particles, exhibit a certain span of the particle size distribution, wherein the span is typically defined as: (span) = (D90 - D10) / D50.

In preferred embodiments of the cosmetic composition according to the invention, the particles within the multitude of solid particles, i.e. the cellodextrin-containing particles, exhibit a span of at least 0.5, or at least 1.0, or at least 1.5, or at least 2.0, or at least 2.5, or at least 3.0, or at least 3.5, or at least 4.0, or at least 4.5, or at least 5.0, or at least 5.5, or at least 6.0, or at least 6.5, or at least 7.0, or at least 7.5, or at least 8.0, or at least 8.5, or at least 9.0, or at least 9.5, or at least 10, determined by laser diffraction analysis according to ISO 13 320.

In preferred embodiments of the cosmetic composition according to the invention, the particles within the multitude of solid particles, i.e. the cellodextrin-containing particles, exhibit a span of at most 10, or at most 9.5, or at most 9.0, or at most 8.5, or at most 8.0, or at most 7.5, or at most 7.0, or at most 6.5, or at most 6.0, or at most 5.5, or at most 5.0, or at most 4.5, or at most 4.0, or at most 3.5, or at most 3.0, or at most 2.5, or at most 2.0, or at most 1.5, or at most 1.0, determined by laser diffraction analysis according to ISO 13 320.

In preferred embodiments of the cosmetic composition according to the invention, at least 10 wt.-%, or at least 20 wt.-%, or at least 30 wt.-%, or at least 40 wt.-%, or at least 50 wt.-%, or at least 60 wt.-%, or at least 70 wt.-%, or at least 80 wt.-%, or at least 90 wt.-% of the particles within the multitude of solid particles, i.e. the cellodextrin-containing particles, have an average particle size, preferably expressed as D50(v) value, within the range of from 0.1 µm to 2500 µm, determined by laser diffraction analysis according to ISO 13 320.

In preferred embodiments of the cosmetic composition according to the invention, at most 90 wt.-%, or at most 80 wt.-%, or at most 70 wt.-%, or at most 60 wt.-%, or at most 50 wt.-%, or at most 40 wt.-%, or at most 30 wt.-%, or at most 20 wt.-%, or at most 10 wt.-% of the particles within the multitude of solid particles, i.e. the cellodextrin-containing particles, have an average particle size, preferably expressed as D50(v) value, within the range of from 0.1 µm to 2500 µm, determined by laser diffraction analysis according to ISO 13 320.

In preferred embodiments of the cosmetic composition according to the invention, at least 10 wt.-%, or at least 20 wt.-%, or at least 30 wt.-%, or at least 40 wt.-%, or at least 50 wt.-%, or at least 60 wt.-%, or at least 70 wt.-%, or at least 80 wt.-%, or at least 90 wt.-% of the particles within the multitude of solid particles, i.e. the cellodextrin-containing particles, have an average particle size, preferably expressed as D50(v) value, within the range of from 1000 µm to 2000 µm, determined by laser diffraction analysis according to ISO 13 320.

In preferred embodiments of the cosmetic composition according to the invention, at most 90 wt.-%, or at most 80 wt.-%, or at most 70 wt.-%, or at most 60 wt.-%, or at most 50 wt.-%, or at most 40 wt.-%, or at most 30 wt.-%, or at most 20 wt.-%, or at most 10 wt.-% of the particles within the multitude of solid particles, i.e. the cellodextrin-containing particles, have an average particle size, preferably expressed as D50(v) value, within the range of from 1000 µm to 2000 µm, determined by laser diffraction analysis according to ISO 13 320.

In preferred embodiments of the cosmetic composition according to the invention, at least 10 wt.-%, or at least 20 wt.-%, or at least 30 wt.-%, or at least 40 wt.-%, or at least 50 wt.-%, or at least 60 wt.-%, or at least 70 wt.-%, or at least 80 wt.-%, or at least 90 wt.-% of the particles within the multitude of solid particles, i.e. the cellodextrin-containing particles, have an average particle size, preferably expressed as D50(v) value, within the range of from 500 µm to 1000 µm, determined by laser diffraction analysis according to ISO 13 320.

In preferred embodiments of the cosmetic composition according to the invention, at most 90 wt.-%, or at most 80 wt.-%, or at most 70 wt.-%, or at most 60 wt.-%, or at most 50 wt.-%, or at most 40 wt.-%, or at most 30 wt.-%, or at most 20 wt.-%, or at most 10 wt.-% of the particles within the multitude of solid particles, i.e. the cellodextrin-containing particles, have an average particle size, preferably expressed as D50(v) value, within the range of from 500 µm to 1000 µm, determined by laser diffraction analysis according to ISO 13 320.

In preferred embodiments of the cosmetic composition according to the invention, at least 10 wt.-%, or at least 20 wt.-%, or at least 30 wt.-%, or at least 40 wt.-%, or at least 50 wt.-%, or at least 60 wt.-%, or at least 70 wt.-%, or at least 80 wt.-%, or at least 90 wt.-% of the particles within the multitude of solid particles, i.e. the cellodextrin-containing particles, have an average particle size, preferably expressed as D50(v) value, within the range of from 200 µm to 500 µm, determined by laser diffraction analysis according to ISO 13 320.

In preferred embodiments of the cosmetic composition according to the invention, at most 90 wt.-%, or at most 80 wt.-%, or at most 70 wt.-%, or at most 60 wt.-%, or at most 50 wt.-%, or at most 40 wt.-%, or at most 30 wt.-%, or at most 20 wt.-%, or at most 10 wt.-% of the particles within the multitude of solid particles, i.e. the cellodextrin-containing particles, have an average particle size, preferably expressed as D50(v) value, within the range of from 200 µm to 500 µm, determined by laser diffraction analysis according to ISO 13 320.

In preferred embodiments of the cosmetic composition according to the invention, at least 10 wt.-%, or at least 20 wt.-%, or at least 30 wt.-%, or at least 40 wt.-%, or at least 50 wt.-%, or at least 60 wt.-%, or at least 70 wt.-%, or at least 80 wt.-%, or at least 90 wt.-% of the particles within the multitude of solid particles, i.e. the cellodextrin-containing particles, have an average particle size, preferably expressed as D50(v) value, within the range of from 75 µm to 200 µm, determined by laser diffraction analysis according to ISO 13 320.

In preferred embodiments of the cosmetic composition according to the invention, at most 90 wt.-%, or at most 80 wt.-%, or at most 70 wt.-%, or at most 60 wt.-%, or at most 50 wt.-%, or at most 40 wt.-%, or at most 30 wt.-%, or at most 20 wt.-%, or at most 10 wt.-% of the particles within the multitude of solid particles, i.e. the cellodextrin-containing particles, have an average particle size, preferably expressed as D50(v) value, within the range of from 75 µm to 200 µm, determined by laser diffraction analysis according to ISO 13 320.

In preferred embodiments of the cosmetic composition according to the invention, at least 10 wt.-%, or at least 20 wt.-%, or at least 30 wt.-%, or at least 40 wt.-%, or at least 50 wt.-%, or at least 60 wt.-%, or at least 70 wt.-%, or at least 80 wt.-%, or at least 90 wt.-% of the particles within the multitude of solid particles, i.e. the cellodextrin-containing particles, have an average particle size, preferably expressed as D50(v) value, within the range (i) of from 0.1 µm to 4.0 µm; or (ii) of from 0.2 µm to 2.0 µm; or (iii) of from 0.3 µm to 1.0 µm; or (iv) of from 0.3 µm to 0.8 µm; determined by laser diffraction analysis according to ISO 13 320.

In preferred embodiments of the cosmetic composition according to the invention, at most 90 wt.-%, or at most 80 wt.-%, or at most 70 wt.-%, or at most 60 wt.-%, or at most 50 wt.-%, or at most 40 wt.-%, or at most 30 wt.-%, or at most 20 wt.-%, or at most 10 wt.-% of the particles within the multitude of solid particles, i.e. the cellodextrin-containing particles, have an average particle size, preferably expressed as D50(v) value, within the range (i) of from 0.1 µm to 4.0 µm; or (ii) of from 0.2 µm to 2.0 µm; or (iii) of from 0.3 µm to 1.0 µm; or (iv) of from 0.3 µm to 0.8 µm; determined by laser diffraction analysis according to ISO 13 320.

The cosmetic composition according to the invention preferably also comprises a lipoid component comprising one or more natural or synthetic lipoids.

In preferred embodiments of the cosmetic composition according to the invention, the weight content of the lipoid component is at least 5 wt.-%, or at least 10 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, in each case relative to the total weight of the cosmetic composition.

In preferred embodiments of the cosmetic composition according to the invention, the weight content of the lipoid component is at most 95 wt.-%, or at most 90 wt.-%, or at most 85 wt.-%, or at most 80 wt.-%, or at most 75 wt.-%, or at most 70 wt.-%, in each case relative to the total weight of the cosmetic composition.

Preferably, the lipoid component comprises or essentially consists of a natural lipoid. Natural lipoids can be selected from lipids of plant origin or animal origin.

Preferred natural lipoids of plant origin are selected from the group consisting of amaranth oil, apricot kernel oil, argan oil, avocado oil, acai oil, babassu oil, baobab oil, bayberry wax, black cumin oil, black currant seed oil, borage seed oil, brazil nut oil, broccoli seed oil, cacao butter, calendula oil, camelina oil, camellia seed oil, carrot seed oil, castor oil, cedarwood seed oil, chamomile oil, chia oil, coconut oil, corn oil, cottonseed oil, cupuacu butter, cranberry seed oil, eucalyptus oil, evening primrose oil, frankincense oil, geranium oil, grape seed oil, groundnut oil, hazelnut oil, hemp oil, illipe butter, Japan wax, jasmine oil, jojoba oil, kokum butter, kukui nut oil, laurel oil, lavender oil, lemongrass oil, linseed oil, macadamia nut oil, mango butter, manketti oil, manila oil, meadowfoam seed oil, milk butter, milk thistle oil, moringa oil, mustard seed oil, olive oil, olus oil, palm oil, palm kernel oil, papaya seed oil, passionfruit seed oil, peach kernel oil, peanut oil, pecan nut oil, peppermint oil, perilla oil, pistachio nut oil, plum kernel oil, pomegranate oil, poppyseed oil, pumpkin seed oil, rapeseed oil, raspberry seed oil, rice bran oil, rose oil, rosehip oil, rosemary oil, sacha inchi oil, safflower oil, sea buckthorn pulp oil, sesame oil, shea butter, soybean oil, St. John's wort oil, sunflower seed oil, squalane oil, sweet almond oil, tall oil, tamanu oil, tea-tree oil, tigernut oil, thyme oil, tung oil, walnut oil, wheat germ oil, and combinations thereof. Hydrocarbon oils of natural origin are for example terpene hydrocarbons such as squalene and squalane.

Preferred natural lipoids of animal origin are marine oils selected from the group consisting of fish oil, whale oil, fish liver oil, and seal oil.

Preferably, the lipoid component comprises or essentially consists of a synthetic lipoid. Synthetic lipoids include but are not limited to
(i) synthetic oils based upon fuel including mineral oil such as white mineral oil, paraffin oil, petroleum jelly oil, petrolatum and the like; liquid paraffins and its derivatives include isoparaffins (e.g. isododecane, isohexadecane, polydecene hydrogenate) and cycloparaffins;
(ii) synthetic fats based upon fuel such as paraffin;
(iii) synthetic silicone oils such as polysiloxanes and their derivatives comprising for example alkyl, alkoxyl or phenyl groups, dimethylpolysiloxane, cyclic silicones, methylphenylpolysiloxane, silicone-glycol copolymers and the like; examples of silicone oils include the polydimethylsiloxanes (dimethicone), amodimethicone, cyclomethicones such as cyclopentasiloxane and cyclohexasiloxane, amino bispropyl dimethicone, aminopropyl dimethicone, amodimethicone hydroxystearate, behenoxy-dimethicone, C₃₀₋₄₅ alkyl dimethicone, C₂₄₋₂₈ alkyl dimethicone, C₃₀₋₄₅ alkyl methicone, cetearyl methicone, cetyl dimethicone, di-methoxysilyl ethylenediaminopropyl dimethicone, hexyl methicone, hydroxypropyldimethicone, stear-amidopropyl dimethicone, stearoxy dimethicone, stearyl methicone, stearyl dimethicone and vinyl dimethicone;
(iv) synthetic silicone fats;
(v) biochemically synthesized oils; or
(vi) biochemically synthesized fats.

Preferably, the lipoid component comprises or essentially consists of a combination of at least two, or at least three, or at least four, or at least five, or at least six of said natural and/or synthetic lipoids.

The cosmetic composition according to the invention may contain a wax. Suitable waxes are the waxes typically used in cosmetic compositions, and may be of natural and/or synthetic origin. Examples of natural waxes are beeswax or cera alba, carnauba wax, candelilla wax, Japan wax, rice wax, waxes deriving from hydrogenated oils such as jojoba oil or sunflower or coconut oils, esters of long chain saturated fatty acids with long chain monoalcohols or their glycerides, such as cetyl palmitate, cetyl stearate, palmitic and stearic triglycerides. Examples of mineral or synthetic waxes are lignite wax, microcrystalline wax, paraffin, ozokerite, ceresin, synthetic beeswax, lanolin and their esters with polyethylene glycols, polyethylene waxes, fatty acid esters having a melting point above 25 °C, cetyl esters, polyamides. Silicone waxes may also be used.

In preferred embodiments, the cosmetic composition according to the invention comprises essentially no water.

In other preferred embodiments, the cosmetic composition according to the invention additionally comprises water.

Suitable methods for determining the water content are known to the skilled person, e.g. Karl-Fischer titration.

In preferred embodiments of the cosmetic composition according to the invention, the water content is at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, in each case relative to the total weight of the cosmetic composition.

In preferred embodiments of the cosmetic composition according to the invention, the water content is at most 85 wt.-%, or at most 80 wt.-%, or at most 75 wt.-%, or at most 70 wt.-%, or at most 65 wt.-%, or at most 60 wt.-%, or at most 55 wt.-%, or at most 50 wt.-%, or at most 45 wt.-%, or at most 40 wt.-%, or at most 35 wt.-%, or at most 30 wt.-%, or at most 25 wt.-%, or most 20 wt.-%, or most 15 wt.-%, or at most 10 wt.-%, or at most 5.0 wt.-%, or at most 1.0 wt.-%, or at most 0.5 wt.-%, in each case relative to the total weight of the cosmetic composition.

The cosmetic composition according to the invention may be a dispersion, an emulsion, a suspension, or any mixture thereof.

According to the IUPAC and for the purpose of the specification, the term "dispersion" or "colloidal dispersion" is defined as a system in which particles of colloidal size of any nature (e.g. solid, liquid or gas) are dispersed in a continuous phase of a different composition (or state) (https://doi.org/10.1351/goldbook.C01174).

According to the IUPAC and for the purpose of the specification, the term "emulsion" is defined as fluid colloidal system in which liquid droplets and/or liquid crystals are dispersed in a liquid. An emulsion is denoted by the symbol O/W if the continuous phase is an aqueous solution and by W/O if the continuous phase is an organic liquid (an 'oil') (https://doi.org/10.1351/goldbook.E02065).

According to the IUPAC and for the purpose of the specification, the term "suspension" is defined as a liquid in which solid particles are dispersed (https://doi.org/10.1351/goldbook. 506198).

In preferred embodiments, the cosmetic composition according to the invention is a water-in-oil emulsion.

In other preferred embodiments, the cosmetic composition according to the invention is an oil-in-water emulsion.

The cosmetic composition according to the invention comprises a surfactant component comprising an anionic surfactant, cationic surfactant, amphoteric surfactant, nonionic surfactant, or any combination thereof.

In preferred embodiments of the cosmetic composition according to the invention, the weight content of the surfactant component is at least 0.1 wt.-%, or at least 1.0 wt.-%, or at least 2.0 wt.-%, or at least 4.0 wt.-%, or at least 6.0 wt.-%, or at least 8.0 wt.-%, or at least 10 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, in each case relative to the total weight of the cosmetic composition.

In preferred embodiments of the cosmetic composition according to the invention, the weight content of the surfactant component is at most 1.0 wt.-%, or at most 2.0 wt.-%, 4.0 wt.-%, or at most 6.0 wt.-%, or at most 8.0 wt.-%, or at most 10 wt.-%, or at most 15 wt.-%, or at most 20 wt.-%, or at most 25 wt.-%, or at most 30 wt.-%, or at most 35 wt.-%, or at most 40 wt.-%, or at most 45 wt.-%, or at most 50 wt.-%, or at most 55 wt.-%, or at most 60 wt.-%, or at most 65 wt.-%, or at most 70 wt.-%, or at most 75 wt.-%, or at most 80 wt.-%, or at most 85 wt.-%, or at most 90 wt.-%, or at most 95 wt.-%, in each case relative to the total weight of the cosmetic composition.

In preferred embodiments of the cosmetic composition according to the invention, the surfactant component comprises or essentially consists of a surfactant selected from the group consisting of
- anionic surfactants which are preferably selected from: sodium laureth sulfate, sodium dodecyl sulfate, sodium lauryl sulfate, ammonium lauryl sulfate, sulfosuccinates (e.g. dioctyl sulfosuccinate, disodium laureth sulfosuccinate, disodium PEG-5 laurylcitrate sulfosuccinate, diethylhexyl sodium sulfosuccinate, disodium ricinoleamido MEA-sulfosuccinate, disodium undecylenamido MEA-sulfosuccinate), acyl methyl taurates (e.g. sodium N-methyl lauroyl taurate), alkylbenzene sulfonates (e.g. dodecylbenzene sulfonate), acyl sarcosinates (e.g. sodium lauroyl sarcosinate), fatty glycerol ether sulfonates (e.g. sodium (3-lauryloxy)-2-hydroxypropane sulfonate), acyl isethionate (e.g. sodium lauroly isethionate), monoglyceride sulfates (e.g. sodium (3-lauroyloxy)-2-hydroxypropyl sulfate), propyl peptide condensates;
- cationic surfactants which are preferably selected from: stearalkonium chlorides (benzyldimethyloctade-cylammonium chloride), dicetyldimonium chloride, docosyltrimethylammonium chloride;
- amphoteric surfactants which are preferably selected from: sodium lauraminopropionate, sodium cocoamphopropionate, cocamidopropyl betaines (e.g. lauramidopropyl betaine), capryl/capramidopropyl betaine, undecylenamidopropyl betaine, disodium cocoamphodiacetate, sodium cocoamphoacetate;
- nonionic surfactants which are preferably selected from: lauramide, lauramide diethanolamine, lauramide oxide, stearamine oxide, poloxamers (e.g. Poloxamer 101, Poloxamer 124), PEG-7 glyceryl cocoate, glyceryl laurate, cocamide diethanolamine, cocamide monoethanolamine;
- biosurfactants which are preferably selected from (i) glycolipids (carbohydrate-lipids) such as rhamnolipids, mannosylerythritol lipids, sophorolipids, cellobioselipids, and the like; (ii) phospholipids; (iii) polyol lipids; (iv) lipoproteins; (v) lipopeptides; (vi) ornithine lipids; (vii) neutral lipids; (viii) aminoacid lipids; (ix) exolipids; (x) liposan; (xi) siderolipids; (xii) protein polyamines diglycosyl diglycerides; (xiii) fimbriae;
and combinations thereof.

In preferred embodiments, the cosmetic composition according to the invention additionally comprises an antioxidant.

Preferably, the antioxidant is selected from tocopherols. Other antioxidants that can be used in the cosmetic composition according to the invention include but are not limited to citric acid, gallic acid, esters of gallic acid, nordihydroguaiaretic acid, thioctic acid (lipoic acid), dihydrolipoic acid, glycolic acid, butylated hydroxytoluene (BHT), butylated hydroxyanisole (BHA), vitamins (e.g. tocopherol, retinol, β-carotene, ascorbic acid) vitamin derivatives (e.g. retinyl acetate, retinyl palmitate, ascorbyl palmitate, magnesium ascorbyl phosphate, tocophoryl acetate), flavonoids, polyphenolic compounds, and the like.

In preferred embodiments, the cosmetic composition according to the invention additionally comprises one or more additives independently of one another selected from the group consisting of acids, fragrances, essential oils, dyes, pigments (e.g. a pigment component), solvents, fillers, thickening agents (e.g. a thickening component), sequestrants, chelating agents, UV absorbers, preservatives, emulsifiers, emollients, and humectants.

In preferred embodiment, the thickening agent comprises or essentially consists of
(i) a natural or synthetic gum, preferably selected from the group consisting of algin, sodium alginate, carrageenan, cellulose gum, guar gum, xanthan gum, karaya gum, gelatin, tragacanth gum, chitosan, acacia gum, and any combination thereof;
(ii) a cellulose ether, preferably selected from the group consisting of methylcellulose, ethylcellulose, hydroxyethyl cellulose, hydroxypropyl cellulose, hydroxypropyl methyl cellulose, carboxymethyl cellulose, and any combination thereof;
(iii) a carbomer (e.g., a crosslinked polyacrylic acid copolymer or homopolymer and copolymers of acrylic acid cross linked with a polyalkenyl polyether);
(iv) an acrylamide polymer, acrylic acid polymer or vinyl polymer, preferably selected from polyvinyl alcohol and polyvinylpyrrolidone;
(v) a polyamine or poly quaternary compound;
(vi) an ethylene oxide polymer;
or any mixtures thereof.

In particularly preferred embodiments of the cosmetic composition according to the invention,
- at least 50 wt.-%, preferably all of the particles within the multitude of solid particles, i.e. the cellodextrin-containing particles, which preferably essentially consist of the cellodextrin component, more preferably of cellobiose, have an average particle size, preferably expressed as D50(v) value, within the range of from 0.1 µm to 4.0 µm, or from 0.2 µm to 2.0 µm, or from 0.3 µm to 1.0 µm, or from 0.3 µm to 0.8 µm, determined by laser diffraction analysis according to ISO 13 320; and/or
- the weight content of the surfactant component is at least 5.0 wt.-%, preferably at least 10 wt.-%, relative to the total weight of the cosmetic composition; and/or
- the cosmetic composition additionally comprises water, wherein the water content is at least 80 wt.-%, relative to the total weight of the cosmetic composition.

In particularly preferred embodiments of the cosmetic composition according to the invention,
- the cellodextrin component comprises or essentially consists of cellobiose; and/or
- the cosmetic composition comprises solid particles comprising or essentially consisting of the cellodextrin component in undissolved form; and/or
- the particles have an average particle size, preferably expressed as D50(v) value determined by laser diffraction analysis according to ISO 13 320, of at most 100 µm, or at most 50 µm, or at most 10 µm, or at most 5.0 µm, or at most 1.0 µm, or at most 0.5 µm, or at most 0.1 µm, or at most 0.05 µm, or at most 0.01 µm, or at most 0.005 µm, or at most 0.001 µm; and/or
- the weight content of the surfactant component is at least 0.1 wt.-%, preferably at least 1.0 wt.-%, more preferably at least 5.0 wt.-%, still more preferably at least 10 wt.-%, relative to the total weight of the cosmetic composition; and/or
- the cosmetic composition additionally comprises water, wherein the water content is at least 20 wt.-%, preferably at least 40 wt.-%, more preferably at least 60 wt.-%, still more preferably at least 80 wt.-%, relative to the total weight of the cosmetic composition; and/or
- the cosmetic composition is selected from liquid soap, foam soap, shower gel and hair shampoo.

For the purpose of the specification opaque means not transparent or translucent.

In preferred embodiments, the cosmetic composition according to the invention has an opacity of at least 25 %, or at least 30 %, or at least 35 %, or at least 40 %, or at least 45 %, or at least 50 %, or at least 55 %, or at least 60 %, or at least 65 %, or at least 70 %, or at least 75 %, or at least 80 %, or at least 85 %, or at least 90 %, or at least 95 %, or at least 100 %, determined by colorimeter measurements.

In preferred embodiments, the cosmetic composition according to the invention has an opacity of at most 95 %, or at most 90 %, or at most 85 %, or at most 80 %, or at most 75 %, or at most 70 %, or at most 65 %, or at most 60 %, or at most 55 %, or at most 50 %, or at most 45 %, or at most 40 %, or at most 35 %, or at most 30 %, or at most 25 %, determined by colorimeter measurements.

In preferred embodiments, the cosmetic composition according to the invention has a turbidity of at least 10 NTU, or at least 20 NTU, or at least 40 NTU, or at least 60 NTU, or at least 80 NTU, or at least 100 NTU, or at least 200 NTU, or at least 400 NTU, or at least 600 NTU, or at least 800 NTU, or at least 1000 NTU, determined by nephelometric analysis.

In preferred embodiments, the cosmetic composition according to the invention has a turbidity of at most 900 NTU, or at most 800 NTU, or at most 600 NTU or at most 400 NTU or at most 200 NTU or at most 100 NTU, or at most 80 NTU or at most 60 NTU or at most 40 NTU or at most 20 NTU or at most 10 NTU, determined by nephelometric analysis.

The cellodextrin component that is present within the cosmetic composition in undissolved form causes the turbidity of the cosmetic composition according to the invention.

In preferred embodiments, the cosmetic composition according to the invention has an intensity of pearlescence, on a scale of 0 to 10, of at least 1, or at least 2, or at least 3, or at least 4, or at least 5, or at least 6, or at least 7, or at least 8, or at least 9, determined in a sensory test according to ISO 13 299.

In preferred embodiments, the cosmetic composition according to the invention has an intensity of pearlescence, on a scale of 0 to 10, of at most 9, or at most 8, or at most 7, or at most 6, or at most 5, or at most 4, or at most 3, or at most 2, or at most 1, determined in a sensory test according to ISO 13 299.

Preferably, the cellodextrin component that is present within the cosmetic composition in undissolved form causes the pearlescence of the cosmetic composition according to the invention.

In preferred embodiments, the cosmetic composition according to the invention has a dynamic viscosity of at least 10 mPa s, or at least 20 mPa s, or at least 40 mPa s, or at least 80 mPa s, or at least 100 mPa s, or at least 200 mPa·s, or at least 400 mPa·s, or at least 600 mPa·s, or at least 800 mPa·s, or at least 1000 mPa·s, determined according to ISO 3 219.

In preferred embodiments, the cosmetic composition according to the invention has a dynamic viscosity of at most 1000 mPa·s, or at most 800 mPa·s, or at most 600 mPa·s, or at most 400 mPa·s, or at most 200 mPa·s, or at most 100 mPa s, or at most 80 mPa·s, or at most 40 mPa s, or at most 20 mPa·s, or at most 10 mPa·s, determined according to ISO 3 219.

In preferred embodiments, the cosmetic composition according to the invention has a dynamic viscosity of at least 2000 mPa s, or at least 4000 mPa s, or at least 6000 mPa s, or at least 8000 mPa s, or at least 10000 mPa s, or at least 11000 mPa s, or at least 12000 mPa s, or at least 13 000 mPa s, or at least 14000 mPa s, or at least 15000 mPa s, determined according to ISO 3 219.

In preferred embodiments, the cosmetic composition according to the invention has a dynamic viscosity of at most 100000000 mPa·s, or at most 10000000 mPa·s, or at most 5 000 000 mPa·s, or at most 1 000000 mPa·s, or at most 500000 mPa·s, or at most 250000 mPa·s, or at most 100000 mPa·s, or at most 50000 mPa·s, or at most 25000 mPa·s, or at most 15000 mPa·s, or at most 14000 mPa·s, or at most 13000 mPa·s, or at most 12000 mPa·s, or at most 11000 mPa·s, or at most 10000 mPa·s, or at most 8000 mPa·s, or at most 4000 mPa·s, or at most 2000 mPa·determined according to ISO 3 219.

In preferred embodiments, the cosmetic composition according to the invention comprises a thickening component. The thickening component is generally used to control or modify the viscosity of the cosmetic composition. This embodiment is particularly relevant when the cosmetic composition according to the invention is e.g. a tooth paste.

In preferred embodiments of the cosmetic composition according to the invention, the weight content of the thickening component is at least 0.01 wt.-%, or at least 0.025 wt.-%, or at least 0.05 wt.-%, or at least 0.075 wt.-%, or at least 0.1 wt.-%, or at least 0.25 wt.-%, or at least 0.5 wt.-%, or at least 0.75 wt.-%, or at least 1.0 wt.-%, or at least 1.25 wt.-%, or at least 1.5 wt.-%, or at least 1.75 wt.-%, or at least 2.0 wt.-%, or at least 2.25 wt.-%, or at least 2.5 wt.-%, or at least 2.75 wt.-%, or at least 3.0 wt.-%, or at least 3.25 wt.-%, or at least 3.5 wt.-%, or at least 3.75 wt.-%, or at least 4.0 wt.-%, or at least 4.25 wt.-%, or at least 4.5 wt.-%, or at least 4.75 wt.-%, or at least 5.0 wt.-%, in each case relative to the total weight of the cosmetic composition.

In preferred embodiments of the cosmetic composition according to the invention, the weight content of the thickening component is at most 2.5 wt.-%, or at most 3.0 wt.-%, or at most 3.5 wt.-%, or at most 4.0 wt.-%, or at most 4.5 wt.-%, or at most 5.0 wt.-%, or at most 6.5 wt.-%, or at most 7.0 wt.-%, or at most 7.5 wt.-%, or at most 8.0 wt.-%, or at most 9.0 wt.-%, or at most 10 wt.-%, or at most 12.5 wt.-%, or at most 15 wt.-%, or at most 17.5 wt.-%, or at most 20 wt.-%, or at most 22.5 wt.-%, or at most 25 wt.-%, in each case relative to the total weight of the cosmetic composition.

Preferably, the thickening component comprises or essentially consists of
(i) a natural or synthetic gum, preferably selected from the group consisting of algin, sodium alginate, carrageenan, cellulose gum, guar gum, xanthan gum, karaya gum, gelatin, tragacanth gum, chitosan, acacia gum, and any combination thereof;
(ii) a cellulose ether, preferably selected from the group consisting of methylcellulose, ethylcellulose, hydroxyethyl cellulose, hydroxypropyl cellulose, hydroxypropyl methyl cellulose, carboxymethyl cellulose, and any combination thereof;
(iii) a carbomer (e.g., a crosslinked polyacrylic acid copolymer or homopolymer and copolymers of acrylic acid cross linked with a polyalkenyl polyether);
(iv) an acrylamide polymer, acrylic acid polymer or vinyl polymer, preferably selected from polyvinyl alcohol and polyvinylpyrrolidone;
(v) a polyamine or polyquarternary compound;
(vi) an ethylene oxide polymer;
and any mixtures thereof.

In preferred embodiments, the cosmetic composition according to the invention comprises a pigment component. The pigment component is generally used to provide chromatic modifications to the cosmetic composition, e.g. to make-ups. This embodiment is particularly relevant when the cosmetic composition according to the invention is a make-up, especially a powdery make-up selected from loose powders and compact powders, face powders, foundations, blushers, and eyeshadows.

In preferred embodiments of the cosmetic composition according to the invention, the weight content of the pigment component is at least 0.01 wt.-%, or at least 0.025 wt.-%, or at least 0.05 wt.-%, or at least 0.075 wt.-%, or at least 0.1 wt.-%, or at least 0.25 wt.-%, or at least 0.5 wt.-%, or at least 0.75 wt.-%, or at least 1.0 wt.-%, or at least 1.25 wt.-%, or at least 1.5 wt.-%, or at least 1.75 wt.-%, or at least 2.0 wt.-%, or at least 2.25 wt.-%, or at least 2.5 wt.-%, or at least 2.75 wt.-%, or at least 3.0 wt.-%, or at least 3.25 wt.-%, or at least 3.5 wt.-%, or at least 3.75 wt.-%, or at least 4.0 wt.-%, or at least 4.25 wt.-%, or at least 4.5 wt.-%, or at least 4.75 wt.-%, or at least 5.0 wt.-%, in each case relative to the total weight of the cosmetic composition.

In preferred embodiments of the cosmetic composition according to the invention, the weight content of the pigment component is at most 2.5 wt.-%, or at most 3.0 wt.-%, or at most 3.5 wt.-%, or at most 4.0 wt.-%, or at most 4.5 wt.-%, or at most 5.0 wt.-%, or at most 6.5 wt.-%, or at most 7.0 wt.-%, or at most 7.5 wt.-%, or at most 8.0 wt.-%, or at most 9.0 wt.-%, or at most 10 wt.-%, or at most 12.5 wt.-%, or at most 15 wt.-%, or at most 17.5 wt.-%, or at most 20 wt.-%, or at most 22.5 wt.-%, or at most 25 wt.-%, in each case relative to the total weight of the cosmetic composition.

Preferably, the pigment component comprises or essentially consists of an organic pigment, a mineral pigment, a nacreous pigment, or any combination thereof.

Preferred organic pigments are precipitated onto a substrate, e.g. aluminum hydroxide or calcium hydroxide, thus forming an insoluble salt.

Preferred examples or mineral pigments include but are not limited to titanium oxides, magnesium oxides, cerium oxides, zinc oxides, chromium oxides, and iron oxides (e.g. yellow iron oxide, red iron oxide, black iron oxide).

Preferred examples or nacreous pigments include but are not limited to guanine (2-amino-6-hydroxy-purine), bismuth oxychloride, titanium dioxide/mica or mica coated by several different layers (silica, iron oxide, titanium dioxide, etc.).

Additional powder components that may be contained in the cosmetic composition according to the invention include, but are not limited to, inorganic powders such as talc, gums, chalk, Fuller's earth, sericite, muscovite, phlogopite, synthetic mica, lepidolite, biotite, lithia mica, vermiculite, aluminum silicate, alkyl and/or trialkyl aryl ammonium smectites, chemically modified magnesium aluminum silicate, organically modified montmorillonite clay, hydrated aluminum silicate, fumed aluminum starch, octenyl succinate, barium silicate, calcium silicate, magnesium silicate, strontium silicate, metal tungstate, magnesium, silica alumina, zeolite, barium sulfate, calcined calcium sulfate (calcined gypsum), calcium phosphate, fluorine apatite, hydroxyapatite, ceramic powder, colloidal silicone dioxide, and boron nitride; organic powders such as polyamide resin powder (nylon powder), cyclodextrin, methyl polymethacrylate powder, copolymer powder of styrene and acrylic acid, benzoguanamine resin powder, polyethylene tetrafluoride) powder, and carboxyvinyl polymer, cellulose powder such as hydroxyethyl cellulose and sodium carboxymethyl cellulose, ethylene glycol monostearate.

The cosmetic composition according to the invention may be available in the form of different product types.

In preferred embodiments, the cosmetic composition according to the invention is a skin or tooth cleansing composition to be rinsed off with water after topical application to the skin or teeth and cleaning.

In preferred embodiments, the cosmetic composition according to the invention is selected from the group consisting of exfoliating scrub soaps, body scrubs, peels (face peels, hand peels, foot peels), handwashing pastes, liquid soaps, shower gels, hair-removal compositions, face masks, and antiacne compositions.

In preferred embodiments, the cosmetic composition according to the invention is a skin care composition to remain on the skin after topical application.

In preferred embodiments, the cosmetic composition according to the invention is selected from hand care composition, body care composition, intimate care compositions, footcare compositions, sunscreen compositions, nailcare compositions, make-up, and repellents.

Another aspect of the invention relates to the use of a cosmetic composition according to the invention as a skin cleansing composition to be rinsed off with water after topical application to the skin and cleaning.

Preferably, the skin cleansing composition is selected from liquid soap, foam soap, shower gel and hair shampoo.

In preferred embodiments, the cosmetic composition according to the invention is used as exfoliating scrub soap, body scrub, peel (face peel, lip peel, hand peel, foot peel), handwashing paste, tooth paste, hair-removal composition, face mask, or antiacne composition and lotions, powders or make-up.

The cosmetic composition according to the invention may be provided in suitable containers that are conventionally used, such as bottles, boxes, packages, dispensers, and the like. The containers can dispense a predetermined amount of the cosmetic composition. In certain aspects, the cosmetic composition is dispensed in a spray, dollop, or liquid.

Another aspect of the invention relates to the use of a cosmetic composition according to the invention as a skin care composition to remain on the skin after topical application.

In preferred embodiments, the cosmetic composition according to the invention is used as hand care composition, body care composition, intimate care composition, footcare composition, tooth paste, sunscreen composition, nailcare composition, make-up or repellent.

### EXAMPLES

The following examples further illustrate the invention but are not to be construed as limiting its scope.

### Body peeling oil gel-scrub and Lip scrubs (Example 1 and Example 2):

General preparation procedure: All exemplified cosmetic compositions in Example 1 and Example 2 have been prepared from three phases (A, B and C) with different ingredients. The ingredients of PHASE A have been mixed sequentially and heated to a temperature of 80 °C under continuous stirring until a clear solution was obtained. Subsequently, the solution was allowed to cool down under continuous stirring until a turbid (hazy) solution was obtained. PHASE B was added and homogenized for 2 minutes using a homogenizer. Finally, PHASE C was added and the components were mixed thoroughly and left untouched. All percentages are based on the total content of the ingredients of PHASE A + PHASE B + PHASE C.

### Example 1:

Seven comparative body peeling oil gel-scrubs (C1 to C7) and one body peeling oil gel-scrub (I1) (not according to the claims) were prepared from the following ingredients

| PHASE | INGREDIENT [wt.-%] | C1 | C2 | C3 | C4 | C5 | C6 | C7 | I1 |
|---|---|---|---|---|---|---|---|---|---|
| A | Coconut oil | 20.0 | 20.0 | 20.0 | 20.0 | 20.0 | 20.0 | 20.0 | 20.0 |
| | Sunflower oil | 14.0 | 14.0 | 14.0 | 14.0 | 14.0 | 14.0 | 14.0 | 14.0 |
| | Avocado oil | 10.0 | 10.0 | 10.0 | 10.0 | 10.0 | 10.0 | 10.0 | 10.0 |
| | Rice bran oil | 10.0 | 10.0 | 10.0 | 10.0 | 10.0 | 10.0 | 10.0 | 10.0 |
| | Castor oil | 10.0 | 10.0 | 10.0 | 10.0 | 10.0 | 10.0 | 10.0 | 10.0 |
| | Grapeseed oil | 10.0 | 10.0 | 10.0 | 10.0 | 10.0 | 10.0 | 10.0 | 10.0 |
| | Hydrogenated vegetable oil | 5.0 | 5.0 | 5.0 | 5.0 | 5.0 | 5.0 | 5.0 | 5.0 |
| | Tocopherol and sunflower seed oil | 0.5 | 0.5 | 0.5 | 0.5 | 0.5 | 0.5 | 0.5 | 0.5 |
| B | Fragrance | 0.5 | 0.5 | 0.5 | 0.5 | 0.5 | 0.5 | 0.5 | 0.5 |
| C | Natural PE beads (500 µm) | 20.0 | - | - | - | - | - | - | - |
| | Shells & Kernels (500 µm) | - | 20.0 | - | - | - | - | - | - |
| | Fruits (500 µm) | - | - | 20.0 | - | - | - | - | - |
| | Seeds (1000 µm) | - | - | - | 20.0 | - | - | - | - |
| | Herbs & Spices (500 µm) | - | - | - | - | 20.0 | - | - | - |
| | Loofah (30 µm) | - | - | - | - | - | 20.0 | - | - |
| | Mineral (500 µm) | - | - | - | - | - | - | 20.0 | - |
| | Cellobiose (D50 150 µm) | - | - | - | - | - | - | - | 20.0 |

### Example 2:

Seven comparative lip scrubs (C8 to C14) and one lip scrub (I2) (not according to the claims) were prepared from the following ingredients:

| PHASE | INGREDIENT [wt.-%] | C8 | C9 | C10 | C11 | C12 | C13 | C14 | I2 |
|---|---|---|---|---|---|---|---|---|---|
| A | Sunflower oil | 15.0 | 15.0 | 15.0 | 15.0 | 15.0 | 15.0 | 15.0 | 15.0 |
| | Grapeseed oil | 15.0 | 15.0 | 15.0 | 15.0 | 15.0 | 15.0 | 15.0 | 15.0 |
| | Avocado oil | 12.0 | 12.0 | 12.0 | 12.0 | 12.0 | 12.0 | 12.0 | 12.0 |
| | Rice bran oil | 12.0 | 12.0 | 12.0 | 12.0 | 12.0 | 12.0 | 12.0 | 12.0 |
| | Castor oil | 12.0 | 12.0 | 12.0 | 12.0 | 12.0 | 12.0 | 12.0 | 12.0 |
| | Hydrogenated vegetable oil | 5.0 | 5.0 | 5.0 | 5.0 | 5.0 | 5.0 | 5.0 | 5.0 |
| | Manila oil | 2.0 | 2.0 | 2.0 | 2.0 | 2.0 | 2.0 | 2.0 | 2.0 |
| | Tocopherol/sunflower seed oil | 0.5 | 0.5 | 0.5 | 0.5 | 0.5 | 0.5 | 0.5 | 0.5 |
| B | Bikira olive/Moringa butter | 6.0 | 6.0 | 6.0 | 6.0 | 6.0 | 6.0 | 6.0 | 6.0 |
| | Fragrance | 0.5 | 0.5 | 0.5 | 0.5 | 0.5 | 0.5 | 0.5 | 0.5 |
| C | Natural PE beads (300 µm) | 20.0 | - | - | - | - | - | - | - |
| | Shells & Kernels (200 µm) | - | 20.0 | - | - | - | - | - | - |
| | Fruits (200 µm) | - | - | 20.0 | - | - | - | - | - |
| | Seeds (1000 µm) | - | - | - | 20.0 | - | - | - | - |
| | Herbs & Spices (200 µm) | - | - | - | - | 20.0 | - | - | - |
| | Loofah (20 µm) | - | - | - | - | - | 20.0 | - | - |
| | Mineral (100 µm) | - | - | - | - | - | - | 20.0 | - |
| | Cellobiose (D50 150 µm) | - | - | - | - | - | - | - | 20.0 |

The cosmetic compositions were tested and evaluated in a panel test with 12 panelists (volunteers). The two different kinds of water-free cosmetic compositions (body peeling oil gel-scrubs of Example 1 and lip scrubs of Example 2) were tested for abrasive action and application performance of cellobiose powders during the sensory test on human skin. Assessment was made by means of a questionnaire.

The test criteria were established by the analyst and were described as follows:
(A) When rinsing off with water: How easy and how fast it is to completely rinse-off the product after application;
(B) After rinsing and pat dry: Which product is preferred in terms of effect on skin/skin feel?

The results of the test are compiled in the following tables:
Criterium (A): Average response of the volunteer after rinsing their skin with water:

| *When rinsing with water* | BODY PEELING OIL GEL-SCRUB | | | LIP SCRUB | | |
|---|---|---|---|---|---|---|
| | I1 | C1 to C7 | same | I2 | C8 to C14 | same |
| Which one is easier to rinse with water? | 62% | 22% | 16% | 54% | 29% | 17% |
| Which one is faster to rinse with water? | 63% | 21% | 16% | 54% | 29% | 17% |

Criterium (B): Average response of the volunteer to the after rinsing and pat dry the skin area:

| *After rinsing and pat dry* | BODY PEELING OIL GEL-SCRUB | | | LIP SCRUB | | |
|---|---|---|---|---|---|---|
| After application on skin, which one do you prefer in terms of effect on skin/skin feel? | | I1 | Ci | | I2 | Ci |
| | I1 vs. C1 | 33% | 67% | I2 vs. C8 | **67%** | 33% |
| | I1 vs. C2 | 22% | 78% | I2 vs. C9 | 11% | 89% |
| | I1 vs. C3 | 33% | 67% | I2 vs. C10 | **67%** | 33% |
| | I1 vs. C4 | **67%** | 33% | I2 vs. C11 | **56%** | 33% |
| | I1 vs. C5 | **78%** | 22% | I2 vs. C12 | **78%** | 22% |
| | I1 vs. C6 | **67%** | 33% | I2 vs. C13 | **56%** | 44% |
| | I1 vs. C7 | **56%** | 44% | I2 vs. C14 | **25%** | 75% |
| | | | | | | |
| **TOTAL VOTES** | | **57%** | **43%** | | **71%** | **29%** |

As shown by the results given in the table above, the majority of volunteers had a clear preference for the peeling product containing cellobiose, when the they were asked for their favorite product with regard to the effect on skin. In case of the body peeling oil gel-scrub product, the inventive formulation (I1 vs. C1-C7), was preferred in 4 of 7 cases (57%). In case of the lip scrub product, the inventive formulation (I2 vs. C8-C14) was preferred in 5 of 7 cases (71%).

Four comparative scrubs based upon natural PE beads (C1/C8), shells & kernels (C2/C9), fruits (C3/C10) and herbs & spices (C5/C12) were compared against the two inventive scrubs I1 and I2, respectively, during application massage over skin. These results are compiled in the following table (not the same means better):

| Result Yes | C1/C8 | C2/C9 | C3/C10 | C5/C12 |
|---|---|---|---|---|
| Do granules have same feel? | 44% | 22% | 22% | 22% |
| Do granules have same abrasive/scrubbing effect? | 33% | 11% | 22% | 22% |

It has been shown by the results of the above panel test that the cosmetic compositions according to the invention are advantageous over cosmetic compositions known from the prior art.

Cellobiose powder is easy and quickly to rinse-off and leaves a good skin feel after use based from the volunteer's response in comparison to other scrubbing agents available in the market.

Compared to other scrubs such as kernels or minerals the clear white/light color of the products containing cellobiose can be considered a marketing advantage.

### Liquid handwash soaps (Example 3 and Example 4):

General preparation procedure: All exemplified liquid handwash soap compositions were prepared from three different types of liquid handwash soaps A, B and C with clear appearance. For reference purposes, a liquid handwash soap D with creamy white appearance was used. Liquid handwash soaps type A to D are commercially available, e.g. type A as Palmolive^{®} *"Aquarium",* type B as Frosch^{®} *"Sensitiv Seife",* type C as Balea^{®} *"Milde Seife Pflege* & *Hygiene"* and type D as Sagrotan^{®} *"Sanft zur Haut Stark gegen Bakterien".*

Composition of the commercially available liquid hand soaps A, B, C and D used for the experiments:

| A: Liquid handwash soap (clear appearance) | B: Liquid handwash soap (clear appearance) | C: Liquid handwash soap (creamy white appearance) | D: Liquid handwash soap (creamy white appearance) |
|---|---|---|---|
| Aqua | Aqua | Aqua | Aqua |
| Sodium Laureth Sulfate | Sodium Laureth Sulfate | Sodium Laureth Sulfate | Sodium Laureth Sulfate |
| Sodium Chloride | Sodium Chloride | Sodium Chloride | Sodium Chloride |
| Parfum | Parfum | Parfum | Parfum |
| Sodium Benzoate | Sodium Benzoate | Sodium Benzoate | Sodium Benzoate |
| Citric Acid | Citric Acid | Citric Acid | Citric Acid |
| CocamidopropyBetaine | CocamidopropBetaine | Cocamidopropyl Betaine | |
| | Glycerin | Glycerin | Glycerin |
| Cocamide MEA^{[1]} | | | Cocamide MEA^{[1]} |
| Tetrasodium EDTA^{[2]} | | | Tetrasodium EDTA^{[2]} |
| | Lactic Acid | Lactic Acid | |
| Sodium Salicylate | | | Salicylic Acid |
| | Coco-Glucoside | Coco-Glucoside | |
| | Glyceryl Oleate | Glyceryl Oleate | |
| | | Sodium Hydroxide | Sodium Hydroxide |
| | | Potassium Sorbate | Potassium Sorbate |
| | | Tocopherol | Tocopheryl Acetate |
| CI 17200^{[3]} | | Tetrasodium Glutamate Diacetate | Styrene/Acrylates Copolymer |
| | | | Magnesium Nitrate |
| | | Hydrogenated Vegetable Glycerides Citrate | Magnesium Chloride |
| | | | Methylchloroisothiazolinone |
| CI 42090^{[4]} | | Ascorbyl Palmitate | |
| | | Lecithin | Methylisothiazolinone |
| | | Benzyl Alcohol | Ammonium Lauryl Sulfate |
| | | Panthenol | Aloe Barbadensis Leaf Juice |

| | | | |
|---|---|---|---|
| ^{[1]} MEA = Monoethanolamine; ^{[2]} EDTA = Ethylenediaminetetraacetic acid; ^{[3]} CI 17200 = Disodium 5-amino-4-hydroxy-3-(phenylazo)naphthalene-2,7-disulphonate; ^{[4]} CI 42090 = Dihydrogen (ethyl)[4-[4-[ethyl(3-sulphonato-benzyl)]amino]-2'-sulphonatobenzhydrylidene]cyclohexa-2,5-dien-1-ylidene](3-sulphonatobenzyl)ammonium, disodium salt | | | |

### Example 3:

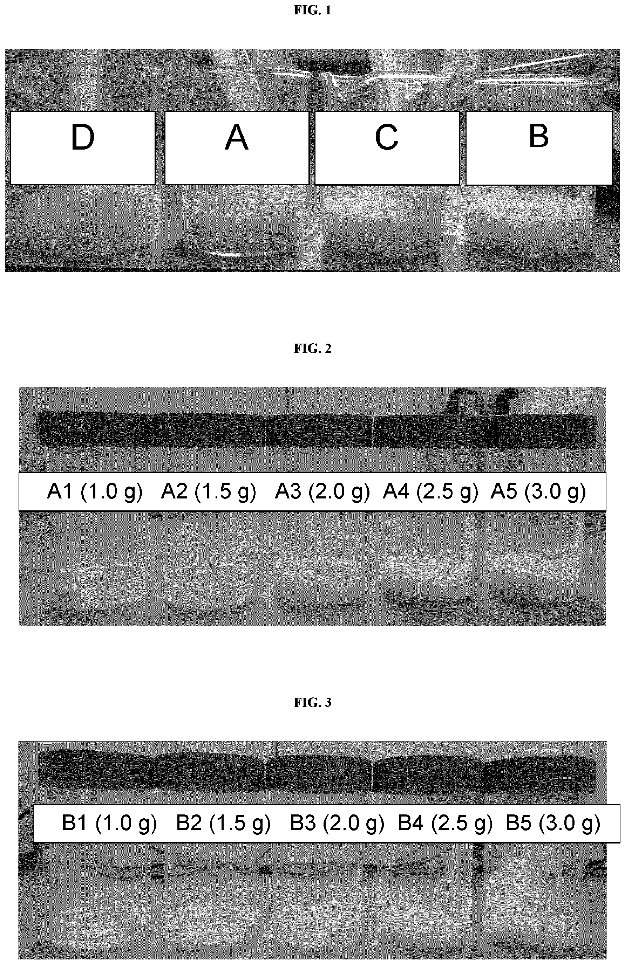

To 20 g of liquid handwash soap A, B and C, respectively, 2 g of cellobiose was added. Subsequently, the mixture was stirred at room temperature for 2.5 h using a magnetic stirrer. Liquid handwash soap D was used as a reference example for a liquid handwash soap with creamy white appearance. Fig. 1 shows that liquid handwash soaps A, B and C which have been mixed with 2.0 g cellobiose have a brighter and more homogenous looking creamy white appearance than reference example liquid handwash soap D.

### Example 4:

General preparation procedure: Overall, 10 samples have been prepared: 5 samples of commercial liquid handwash soap A and 5 samples of commercial liquid handwash soap B have been prepared. Therefore, for each of the test series with the liquid handwash soaps A and B, respectively, a quantity of five times 20 g of the liquid handwash soap was filled into plastic beakers equipped with screw caps. Subsequently, a different amount of cellobiose (1 g; 1.5 g; 2.0 g; 2.5 g; and 3.0 g) was then added to each, the beaker was sealed, and the mixtures were stirred for 2.5 hat room temperature with a magnetic stirrer. Following, all samples were kept in the sealed beakers for a period of 1 day to 3 days at room temperature. Further, after 1 day and 3 days, respectively, a 1 mL sample was taken of each mixture, transferred to a centrifuge tube and centrifuged at 14.000x g for 30 min.

Samples of commercially available liquid handwash soaps A and B with different amounts of cellobiose added:

| INGREDIENT [g] / SAMPLE | A1 | A2 | A3 | A4 | A5 | B1 | B2 | B3 | B4 | B5 |
|---|---|---|---|---|---|---|---|---|---|---|
| Liquid handwash soap A | 20.0 | 20.0 | 20.0 | 20.0 | 20.0 | - | - | - | - | - |
| Liquid handwash soap B | - | - | - | - | - | 20.0 | 20.0 | 20.0 | 20.0 | 20.0 |
| Cellobiose | 1 | 1.5 | 2.0 | 2.5 | 3.0 | 1 | 1.5 | 2.0 | 2.5 | 3.0 |
| Total | 21.0 | 21.5 | 22.0 | 22.5 | 23.0 | 21.0 | 21.5 | 22.0 | 22.5 | 23.0 |
| Weight-% of cellobiose^{[5]} | \| 4.8 | \| 7.0 | \| 9.1 | \| 11.1 | I 13.0 | 4.8 | 7.0 | 9.1 | 1 11.1 | 13.0 |

| | | | | | | | | | | |
|---|---|---|---|---|---|---|---|---|---|---|
| ^{[5]} With respect to the total weight of the cosmetic composition. | | | | | | | | | | |

Figures 2 and 3 show photographs of samples A1 to A5 after 2.5 h (Fig. 2) and 3 days (Fig. 3) of stirring.

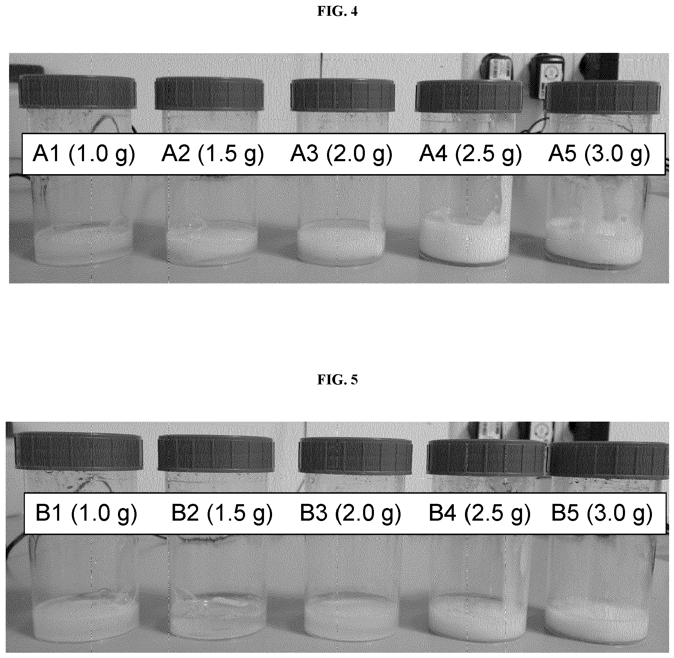

Figures 4 and 5 show photographs of samples B1 to B5 after 2.5 h (Fig. 4) and 3 days (Fig. 5) of stirring

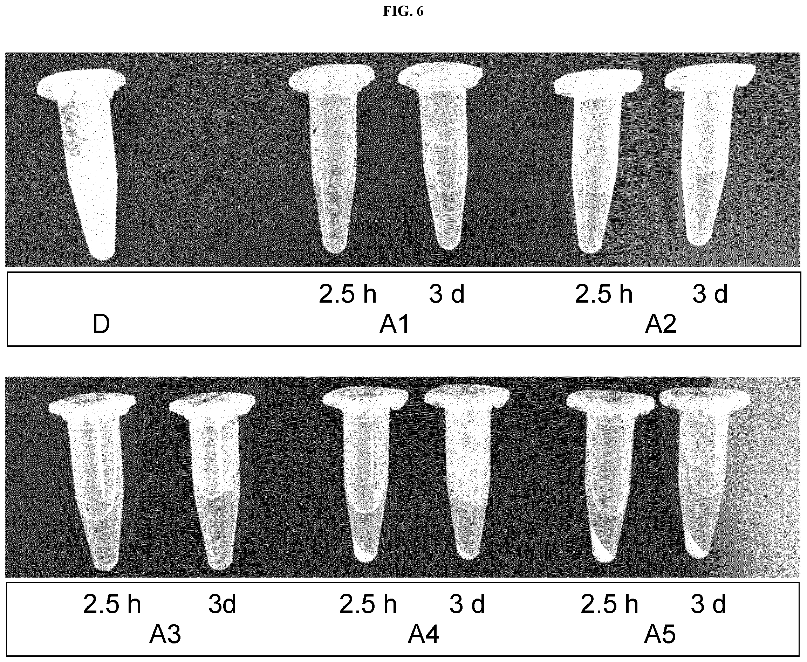

Figure 6 shows photographs of centrifuged samples A1 to A5 and reference liquid handwash soap D.

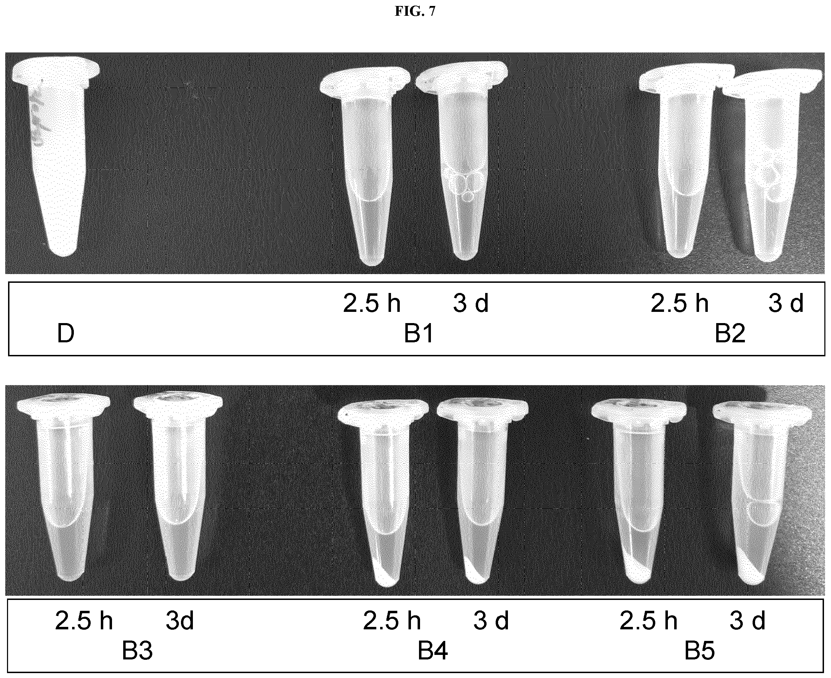

Figure 7 shows photographs of centrifuged samples B1 to B5 and reference liquid handwash soap D.

It has been shown by the results of the above Example 3 and Example 4 that the cosmetic compositions according to the invention are advantageous over cosmetic compositions known from the prior art, e.g. the liquid handwash soaps with cellobiose have a brighter and more homogenous looking creamy white appearance than comparable liquid handwash soaps known from the prior art.

The project leading to this patent application has received funding from the European Union's Horizon 2020 research and innovation program under grant agreement No. 761030.

## Claims

1. A cosmetic composition comprising a cellodextrin component comprising
- a cellosaccharide selected from the group consisting of cellobiose, cellotriose, cellotetraose, cellopentaose, cellohexaose, celloheptaose, cellooctaose, cellononaose, cellodecaose, celloundecaose, cellododecaose, and combinations thereof; and
- a surfactant component comprising an anionic surfactant, cationic surfactant, amphoteric surfactant, nonionic surfactant, or any combination thereof;
wherein at least a portion of said cellodextrin component is present within the cosmetic composition in undissolved form; and
wherein the cosmetic composition has a turbidity of at least 5 NTU, determined by nephelometric analysis.

2. The cosmetic composition according to claim 1, wherein, the total content of the cellodextrin component is at least 0.01 wt.-%, or at least 0.1 wt.-%, or at least 1.0 wt.-%, preferably at least 5.0 wt.-%, more preferably at least 10 wt.-%, still more preferably at least 15 wt.-%, yet more preferably at least 20 wt.-%, even more preferably at least 25 wt.-%, yet more preferably at least 30 wt.-%, and most preferably at least 35 wt.-%, in each case relative to the total weight of the cosmetic composition.

3. The cosmetic composition according to any of the preceding claims, wherein the portion of the cellodextrin component that is present in undissolved form amounts to at least 5.0 wt.-%, or at least 10 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, in each case of the total amount of the cellodextrin component that is contained in the cosmetic composition.

4. The cosmetic composition according to any of the preceding claims, wherein the cosmetic composition comprises a multitude of solid particles, wherein at least a portion of the multitude of solid particles comprises at least a portion of the cellodextrin component.

5. The cosmetic composition according to claim 4, wherein the solid particles which comprise at least a portion of the cellodextrin component essentially consist of the cellodextrin component.

6. The cosmetic composition according to claim 4 or 5, wherein substantially the total amount of the cellodextrin component which is comprised in the cosmetic composition is comprised in the multitude of solid particles.

7. The cosmetic composition according to any of claims 4 to 6, wherein the particles within the multitude of solid particles (i.e. the cellodextrin-containing particles) have a D50(v) value of at most 100 µm, or at most 50 µm, or at most 10 µm, or at most 5.0 µm, or at most 1.0 µm, or at most 0.5 µm, or at most 0.1 µm, or at most 0.05 µm, or at most 0.01 µm, or at most 0.005 µm, or at most 0.001 µm, determined by laser diffraction analysis according to ISO 13 320.

8. The cosmetic composition according to any of the preceding claims, wherein the weight content of the surfactant component is at least 0.1 wt.-%, or at least 1.0 wt.-%, or at least 2.0 wt.-%, or at least 4.0 wt.-%, or at least 6.0 wt.-%, or at least 8.0 wt.-%, or at least 10 wt.-%, or at least 15 wt.-%, or at least 20 wt.-%, or at least 25 wt.-%, or at least 30 wt.-%, or at least 35 wt.-%, or at least 40 wt.-%, or at least 45 wt.-%, or at least 50 wt.-%, or at least 55 wt.-%, or at least 60 wt.-%, or at least 65 wt.-%, or at least 70 wt.-%, or at least 75 wt.-%, or at least 80 wt.-%, or at least 85 wt.-%, or at least 90 wt.-%, or at least 95 wt.-%, in each case relative to the total weight of the cosmetic composition.

9. The cosmetic composition according to any of the preceding claims, wherein the weight content of the surfactant component is at most 1.0 wt.-%, or at most 2.0 wt.-%, 4.0 wt.-%, or at most 6.0 wt.-%, or at most 8.0 wt.-%, or at most 10 wt.-%, or at most 15 wt.-%, or at most 20 wt.-%, or at most 25 wt.-%, or at most 30 wt.-%, or at most 35 wt.-%, or at most 40 wt.-%, or at most 45 wt.-%, or at most 50 wt.-%, or at most 55 wt.-%, or at most 60 wt.-%, or at most 65 wt.-%, or at most 70 wt.-%, or at most 75 wt.-%, or at most 80 wt.-%, or at most 85 wt.-%, or at most 90 wt.-%, or at most 95 wt.-%, in each case relative to the total weight of the cosmetic composition.

10. The cosmetic composition according to any of the preceding claims, wherein the surfactant component comprises or essentially consists of a surfactant selected from the group consisting of
- anionic surfactants which are preferably selected from: sodium laureth sulfate, sodium dodecyl sulfate, sodium lauryl sulfate, ammonium lauryl sulfate, sulfosuccinates (e.g. dioctyl sulfosuccinate, disodium laureth sulfosuccinate, disodium PEG-5 laurylcitrate sulfosuccinate, diethylhexyl sodium sulfosuccinate, disodium ricinoleamido MEA-sulfosuccinate, disodium undecylenamido MEA-sulfosuccinate), acyl methyl taurates (e.g. sodium N-methyl lauroyl taurate), alkylbenzene sulfonates (e.g. dodecylbenzene sulfonate), acyl sarcosinates (e.g. sodium lauroyl sarcosinate), fatty glycerol ether sulfonates (e.g. sodium (3-lauryloxy)-2-hydroxypropane sulfonate), acyl isethionate (e.g. sodium lauroly isethionate), monoglyceride sulfates (e.g. sodium (3-lauroyloxy)-2-hydroxypropyl sulfate), propyl peptide condensates;
- cationic surfactants which are preferably selected from: stearalkonium chlorides (benzyldimethyloctadecylammonium chloride), dicetyldimonium chloride, docosyltrimethylammonium chloride;
- amphoteric surfactants which are preferably selected from: sodium lauraminopropionate, sodium cocoamphopropionate, cocamidopropyl betaines (e.g. lauramidopropyl betaine), capryl/capramidopropyl betaine, undecylenamidopropyl betaine, disodium cocoamphodiacetate, sodium cocoamphoacetate;
- nonionic surfactants which are preferably selected from: lauramide, lauramide diethanolamine, lauramide oxide, stearamine oxide, poloxamers (e.g. Poloxamer 101, Poloxamer 124), PEG-7 glyceryl cocoate, glyceryl laurate, cocamide diethanolamine, cocamide monoethanolamine;
- biosurfactants which are preferably selected from (i) glycolipids (carbohydrate-lipids) such as rhamnolipids, mannosylerythritol lipids, sophorolipids, cellobioselipids, and the like; (ii) phospholipids; (iii) polyol lipids; (iv) lipoproteins; (v) lipopeptides; (vi) ornithine lipids; (vii) neutral lipids; (viii) aminoacid lipids; (ix) exolipids; (x) liposan; (xi) siderolipids; (xii) protein polyamines diglycosyl diglycerides; (xiii) fimbriae; and combinations thereof.

11. The cosmetic composition according to any of the preceding claims, wherein
- the cellodextrin component comprises or essentially consists of cellobiose;
- the cosmetic composition comprises solid particles comprising or essentially consisting of the cellodextrin component in undissolved form;
- the particles have an average particle size, preferably expressed as D50(v) value determined by laser diffraction analysis according to ISO 13 320, of at most 100 µm, or at most 50 µm, or at most 10 µm, or at most 5.0 µm, or at most 1.0 µm, or at most 0.5 µm, or at most 0.1 µm, or at most 0.05 µm, or at most 0.01 µm, or at most 0.005 µm, or at most 0.001 µm;
- the weight content of the surfactant component is at least 0.1 wt.-%, preferably at least 1.0 wt.-%, more preferably at least 5.0 wt.-%, still more preferably at least 10 wt.-%, relative to the total weight of the cosmetic composition;
- the cosmetic composition additionally comprises water, wherein the water content is at least 20 wt.-%, preferably at least 40 wt.-%, more preferably at least 60 wt.-%, still more preferably at least 80 wt.-%, relative to the total weight of the cosmetic composition;
- the cosmetic composition is selected from liquid soap, foam soap, shower gel and hair shampoo.

12. The cosmetic composition according to any of the preceding claims, wherein the weight content of cellobiose is at least 1.0 wt.-%, or at least 2.0 wt.-%, or at least 3.0 wt.-%, or at least 4.0 wt.-%, or at least 5.0 wt.-%, or at least 7.5 wt.-%, or at least 10 wt.-%, or at least 12.5 wt.-%, or at least 15 wt.-%, or at least 17.5 wt.-%, or at least 20 wt.-%, in each case relative to the total weight of the cosmetic composition.

13. The cosmetic composition according to any of the preceding claims, which additionally comprises an antioxidant.

14. The cosmetic composition according to any of the preceding claims, which has an opacity of at least 25 %, or at least 30 %, or at least 35 %, or at least 40 %, or at least 45 %, or at least 50 %, or at least 55 %, or at least 60 %, or at least 65 %, or at least 70 %, or at least 75 %, or at least 80 %, or at least 85 %, or at least 90 %, or at least 95 %, or at least 100 %, determined by colorimeter measurements.

15. The cosmetic composition according to any of the preceding claims, which has an intensity of pearlescence, on a scale of 0 to 10, of at least 1, or at least 2, or at least 3, or at least 4, or at least 5, or at least 6, or at least 7, or at least 8, or at least 9, determined in a sensory test according to ISO 13 299.

## Patentansprüche

1. Eine kosmetische Zusammensetzung, umfassend eine Cellodextrin-Komponente, die Folgendes umfasst
- ein Cellosaccharid, das ausgewählt wird aus der Gruppe bestehend aus Cellobiose, Cellotriose, Cellotetraose, Cellopentaose, Cellohexaose, Celloheptaose, Cellooctaose, Cellononaose, Cellodecaose, Celloundecaose, Cellododecaose und Kombinationen davon; und
- eine Tensidkomponente, die ein anionisches Tensid, ein kationisches Tensid, ein amphoteres Tensid, ein nichtionisches Tensid oder eine Kombination davon umfasst;
wobei mindestens ein Teil der Cellodextrin-Komponente in der kosmetischen Zusammensetzung in ungelöster Form vorhanden ist; und
wobei die kosmetische Zusammensetzung eine Trübung von mindestens 5 NTU, bestimmt durch nephelometrische Analyse, aufweist.

2. Die kosmetische Zusammensetzung gemäß Anspruch 1, wobei der Gesamtgehalt der Cellodextrin-Komponente mindestens 0,01 Gew.-% oder mindestens 0,1 Gew.-% oder mindestens 1,0 Gew.-%, vorzugsweise mindestens 5,0 Gew.-%, noch bevorzugter mindestens 10 Gew.-%, noch bevorzugter mindestens 15 Gew.-%, noch bevorzugter mindestens 20 Gew.-%, noch bevorzugter mindestens 25 Gew.-% und noch bevorzugter mindestens 30 Gew.-%, und am meisten bevorzugt mindestens 35 Gew.-%, jeweils bezogen auf das Gesamtgewicht der kosmetischen Zusammensetzung, beträgt.

3. Die kosmetische Zusammensetzung gemäß einem der vorhergehenden Ansprüche, wobei der Anteil der Cellodextrin-Komponente, der in ungelöster Form vorliegt, mindestens 5,0 Gew.-% oder mindestens 10 Gew.-% oder mindestens 15 Gew.-% oder mindestens 20 Gew.-% oder mindestens 25 Gew.-% oder mindestens 30 Gew.-% oder mindestens 35 Gew.-% oder mindestens 40 Gew.-% oder mindestens 45 Gew.-% oder mindestens 50 Gew.-% oder mindestens 55 Gew.-% oder mindestens 60 Gew.-% oder mindestens 65 Gew.-% oder mindestens 70 Gew.-% oder mindestens 75 Gew.-% oder mindestens 80 Gew.-% oder mindestens 85 Gew.-% oder mindestens 90 Gew.-% oder mindestens 95 Gew.-%, jeweils bezogen auf die Gesamtmenge der in der kosmetischen Zusammensetzung enthaltenen Cellodextrin-Komponente, beträgt.

4. Die kosmetische Zusammensetzung gemäß einem der vorhergehenden Ansprüche, wobei die kosmetische Zusammensetzung eine Vielzahl von festen Teilchen umfasst, wobei mindestens ein Teil der Vielzahl von festen Teilchen mindestens einen Teil der Cellodextrin-Komponente umfasst.

5. Die kosmetische Zusammensetzung gemäß Anspruch 4, wobei die festen Teilchen, die mindestens einen Teil der Cellodextrin-Komponente umfassen, im Wesentlichen aus der Cellodextrin-Komponente bestehen.

6. Die kosmetische Zusammensetzung gemäß Anspruch 4 oder 5, wobei im Wesentlichen die Gesamtmenge der Cellodextrin-Komponente, die in der kosmetischen Zusammensetzung enthalten ist, in der Vielzahl der festen Teilchen enthalten ist.

7. Die kosmetische Zusammensetzung gemäß einem der Ansprüche 4 bis 6, wobei die Teilchen innerhalb der Vielzahl fester Teilchen (d.h. die cellodextrinhaltigen Teilchen) einen D50(v)-Wert von höchstens 100 µm, oder höchstens 50 µm, oder höchstens 10 µm, oder höchstens 5,0 µm, oder höchstens 1,0 µm, oder höchstens 0,5 µm, oder höchstens 0,1 µm, oder höchstens 0,05 µm, oder höchstens 0,01 µm, oder höchstens 0,005 µm, oder höchstens 0,001 µm, bestimmt durch Laserbeugungsanalyse gemäß ISO 13 320, aufweisen.

8. Die kosmetische Zusammensetzung gemäß einem der vorhergehenden Ansprüche, wobei der Gewichtsgehalt der Tensidkomponente mindestens 0,1 Gew.-% oder mindestens 1,0 Gew.-% oder mindestens 2,0 Gew.-% oder mindestens 4,0 Gew.-% oder mindestens 8,0 Gew.-% oder mindestens 10 Gew.-% oder mindestens 15 Gew.-% oder mindestens 20 Gew.-% oder mindestens 25 Gew.-% oder mindestens 30 Gew.-%, oder mindestens 35 Gew.-%, oder mindestens 40 Gew.-%, oder mindestens 45 Gew.-%, oder mindestens 50 Gew.-%, oder mindestens 55 Gew.-%, oder mindestens 60 Gew.-%, oder mindestens 65 Gew.-%, oder mindestens 70 Gew.-%, oder mindestens 75 Gew.-%, oder mindestens 80 Gew.-%, oder mindestens 85 Gew.-%, oder mindestens 90 Gew.-%, oder mindestens 95 Gew.-%, jeweils bezogen auf das Gesamtgewicht der kosmetischen Zusammensetzung, beträgt.

9. Die kosmetische Zusammensetzung gemäß einem der vorhergehenden Ansprüche, wobei der Gewichtsgehalt der Tensidkomponente höchstens 1,0 Gew.-% oder höchstens 2,0 Gew.-%, 4,0 Gew.-% oder höchstens 6,0 Gew.-% oder höchstens 8,0 Gew.-% oder höchstens 10 Gew.-% oder höchstens 15 Gew.-% oder höchstens 20 Gew.-% oder höchstens 25 Gew.-% oder höchstens 30 Gew.-% oder höchstens 35 Gew.-%, oder höchstens 40 Gew.-%, oder höchstens 45 Gew.-%, oder höchstens 50 Gew.-%, oder höchstens 55 Gew.-%, oder höchstens 60 Gew.-%, oder höchstens 65 Gew.-%, oder höchstens 70 Gew.-%, oder höchstens 75 Gew.-%, oder höchstens 80 Gew.-%, oder höchstens 85 Gew.-%, oder höchstens 90 Gew.-%, oder höchstens 95 Gew.-%, jeweils bezogen auf das Gesamtgewicht der kosmetischen Zusammensetzung, beträgt.

10. Die kosmetische Zusammensetzung gemäß einem der vorhergehenden Ansprüche, wobei die Tensidkomponente ein Tensid umfasst oder im Wesentlichen aus einem Tensid besteht, das ausgewählt wird aus der Gruppe bestehend aus
- anionische Tenside, die vorzugsweise ausgewählt werden aus: Natriumlaurethsulfat, Natriumdodecylsulfat, Natriumlaurylsulfat, Ammoniumlaurylsulfat, Sulfosuccinaten (z. B. Dioctylsulfosuccinat, Dinatriumlaurethsulfosuccinat, Dinatrium-PEG-5-Laurylcitrat-Sulfosuccinat, Diethylhexylnatriumsulfosuccinat, Dinatriumricinoleamido MEA-Sulfosuccinat, Dinatriumundecylenamido MEA-Sulfosuccinat), Acylmethyltaurate (z. B. Natrium-N-methyllauroyltaurat), Alkylbenzolsulfonate (z. B. Dodecylbenzolsulfonat), Acylsarkosinate (z. B. Natriumlauroylsarkosinat), Fettglycerinethersulfonate (z. B. Natrium-(3-lauryloxy)-2-hydroxypropansulfonat), Acylisethionat (z. B. Natriumlaurolylisethionat), Monoglyceridsulfate (z. B. Natrium-(3-lauryloxy)-2-hydroxypropylsulfat), Propylpeptidkondensate;
- kationische Tenside, die vorzugsweise ausgewählt werden aus: Stearalkoniumchloriden (Benzyldimethyloctadecylammoniumchlorid), Dicetyldimoniumchlorid, Docosyltrimethylammoniumchlorid;
- amphotere Tenside, die vorzugsweise ausgewählt werden aus: Natriumlauraminopropionat, Natriumcocoamphopropionat, Cocamidopropylbetainen (z. B. Lauramidopropylbetain), Capryl/Capramidopropylbetain, Undecylenamidopropylbetain, Dinatriumcocoamphodiacetat, Natriumcocoamphoacetat;
- nichtionische Tenside, die vorzugsweise ausgewählt werden aus: Lauramid, Lauramid-Diethanolamin, Lauramidoxid, Stearaminoxid, Poloxameren (z. B. Poloxamer 101, Poloxamer 124), PEG-7-Glycerylcocoat, Glyceryllaurat, Cocamid-Diethanolamin, Cocamid-Monoethanolamin;
- Biotenside, die vorzugsweise ausgewählt werden aus (i) Glykolipiden (Kohlenhydratlipiden) wie Rhamnolipiden, Mannosylerythritolipiden, Sophorolipiden, Cellobioselipiden und dergleichen; (ii) Phospholipiden; (iii) Polyolipiden; (iv) Lipoproteinen; (v) Lipopeptide; (vi) Ornithinlipide; (vii) neutrale Lipide; (viii) Aminosäurelipide; (ix) Exolipide; (x) Liposan; (xi) Siderolipide; (xii) Proteinpolyamindiglycosyldiglyceride; (xiii) Fimbrien; und Kombinationen davon.

11. Die kosmetische Zusammensetzung gemäß einem der vorhergehenden Ansprüche, wobei
- der Cellodextrinbestandteil Cellobiose umfasst oder im Wesentlichen aus Cellobiose besteht;
- die kosmetische Zusammensetzung feste Teilchen, die die Cellodextrin-Komponente in ungelöster Form enthalten oder im Wesentlichen aus dieser bestehen, umfasst;
- die Teilchen eine durchschnittliche Teilchengröße, vorzugsweise ausgedrückt als D50(v)-Wert, bestimmt durch Laserbeugungsanalyse gemäß ISO 13 320, von höchstens 100 µm oder höchstens 50 µm oder höchstens 10 µm oder höchstens 5,0 µm oder höchstens 1,0 µm oder höchstens 0,5 µm oder höchstens 0,1 µm oder höchstens 0,05 µm oder höchstens 0,01 µm oder höchstens 0,005 µm oder höchstens 0,001 µm aufweisen;
- der Gewichtsanteil der Tensidkomponente mindestens 0,1 Gew.-%, vorzugsweise mindestens 1,0 Gew.-%, weiter bevorzugt mindestens 5,0 Gew.-%, noch weiter bevorzugt mindestens 10 Gew.-%, bezogen auf das Gesamtgewicht der kosmetischen Zusammensetzung, beträgt;
- die kosmetische Zusammensetzung zusätzlich Wasser enthält, wobei der Wassergehalt mindestens 20 Gew.-%, vorzugsweise mindestens 40 Gew.-%, noch bevorzugter mindestens 60 Gew.-%, noch bevorzugter mindestens 80 Gew.-%, bezogen auf das Gesamtgewicht der kosmetischen Zusammensetzung, beträgt;
- die kosmetische Zusammensetzung ausgewählt wird aus Flüssigseife, Schaumseife, Duschgel und Haarshampoo.

12. Die kosmetische Zusammensetzung gemäß einem der vorhergehenden Ansprüche, wobei der Gewichtsgehalt an Cellobiose mindestens 1,0 Gew.-% oder mindestens 2,0 Gew.-% oder mindestens 3,0 Gew.-% oder mindestens 4,0 Gew.-% oder mindestens 5,0 Gew.-% oder mindestens 7,5 Gew.-% oder mindestens 10 Gew.-% oder mindestens 12,5 Gew.-% oder mindestens 15 Gew.-% oder mindestens 17,5 Gew.-% oder mindestens 20 Gew.-%, jeweils bezogen auf das Gesamtgewicht der kosmetischen Zusammensetzung, beträgt.

13. Die kosmetische Zusammensetzung gemäß einem der vorhergehenden Ansprüche, die zusätzlich ein Antioxidans enthält.

14. Die kosmetische Zusammensetzung gemäß einem der vorhergehenden Ansprüche, die eine Opazität von mindestens 25 % oder mindestens 30 % oder mindestens 35 % oder mindestens 40 % oder mindestens 45 % oder mindestens 50 % oder mindestens 55 % oder mindestens 60 % oder mindestens 65 % oder mindestens 70 % oder mindestens 75 % oder mindestens 80 % oder mindestens 85 % oder mindestens 90 % oder mindestens 95 % oder mindestens 100 %, bestimmt durch Kolorimeter-Messungen, aufweist.

15. Die kosmetische Zusammensetzung gemäß einem der vorhergehenden Ansprüche, die eine Intensität des Perlglanzes auf einer Skala von 0 bis 10 von mindestens 1 oder mindestens 2 oder mindestens 3 oder mindestens 4 oder mindestens 5 oder mindestens 6 oder mindestens 7 oder mindestens 8 oder mindestens 9, bestimmt in einem sensorischen Test gemäß ISO 13 299, aufweist.

## Revendications

1. Composition cosmétique comprenant un constituant cellodextrine comprenant :
- un cellosaccharide choisi parmi le groupe consistant de cellobiose, cellotriose, cellotétraose, cellopentaose, cellohexaose, celloheptaose, cellooctaose, cellononaose, cellodécaose, celloundécaose, cellododécaose, et en combinaisons de ceux-ci ; et
- un constituant tensioactif comprenant un tensioactif anionique, un tensioactif cationique, un tensioactif amphotère, un tensioactif non ionique ou toute combinaison de ceux-ci ;
dans laquelle au moins une partie dudit constituant cellodextrine est présente dans la composition cosmétique sous forme non dissoute ; et
dans laquelle la composition cosmétique présente une turbidité d'au moins 5 NTU, déterminée par analyse néphélométrique.

2. Composition cosmétique selon la revendication 1, dans laquelle la teneur totale du constituant cellodextrine est d'au moins 0,01 % en poids, ou d'au moins 0,1 % en poids, ou d'au moins 1,0 % en poids, de préférence d'au moins 5,0 % en poids, plus préférentiellement d'au moins 10 % en poids, et encore plus préférentiellement d'au moins 15 % en poids, mais encore plus préférentiellement d'au moins 20 % en poids, encore plus préférentiellement d'au moins 25 % en poids, de manière préférée entre toutes d'au moins 30 % en poids, et le plus préférentiellement d'au moins 35 % en poids, dans chaque cas, par rapport au poids total de la composition cosmétique.

3. Composition cosmétique selon l'une quelconque des revendications précédentes, dans laquelle la partie du constituant cellodextrine qui est présente sous forme non dissoute correspond à au moins 5,0 % en poids, ou au moins 10 % en poids, ou au moins 15 % en poids, ou au moins 20 % en poids, ou au moins 25 % en poids, ou au moins 30 % en poids, ou au moins 35 % en poids, ou au moins 40 % en poids, ou au moins 45 % en poids, ou au moins 50 % en poids, ou au moins 55 % en poids, ou au moins 60 % en poids, ou au moins 65 % en poids, ou au moins 70 % en poids, ou au moins 75 % en poids, ou au moins 80 % en poids, ou au moins 85 % en poids, ou au moins 90 % en poids, ou au moins 95 % en poids, dans chaque cas, de la quantité totale du constituant cellodextrine qui est contenu dans la composition cosmétique.

4. Composition cosmétique selon l'une quelconque des revendications précédentes, dans laquelle la composition cosmétique comprend une multitude de particules solides, dans laquelle au moins une partie de la multitude de particules solides comprend au moins une partie du constituant cellodextrine.

5. Composition cosmétique selon la revendication 4, dans laquelle les particules solides qui comprennent au moins une partie du constituant cellodextrine sont essentiellement constituées du constituant cellodextrine.

6. Composition cosmétique selon la revendication 4 ou 5, dans laquelle sensiblement la quantité totale du constituant cellodextrine qui est compris dans la composition cosmétique est comprise dans la multitude de particules solides.

7. Composition cosmétique selon l'une quelconque des revendications 4 à 6, dans laquelle les particules parmi la multitude de particules solides (c'est-à-dire les particules contenant de la cellodextrine) ont une valeur D50(v) d'au plus 100 µm, ou d'au plus 50 µm, ou d'au plus 10 µm, ou d'au plus 5,0 µm, ou d'au plus 1,0 µm, ou d'au plus 0,5 µm, ou d'au plus 0,1 µm, ou d'au plus 0,05 µm, ou d'au plus 0,01 µm, ou d'au plus 0,005 µm, ou d'au plus 0,001 µm, déterminée par analyse par diffraction laser selon la norme ISO 13 320.

8. Composition cosmétique selon l'une quelconque des revendications précédentes, dans laquelle la teneur en poids du constituant tensioactif est d'au moins 0,1 % en poids, ou d'au moins 1,0 % en poids, ou d'au moins 2,0 % en poids, ou d'au moins 4,0 % en poids, ou d'au moins 6,0 % en poids, ou d'au moins 8,0 % en poids, ou d'au moins 10 % en poids, ou d'au moins 15 % en poids, ou d'au moins 20 % en poids, ou d'au moins 25 % en poids, ou d'au moins 30 % en poids, ou d'au moins 35 % en poids, ou d'au moins 40 % en poids, ou d'au moins 45 % en poids, ou d'au moins 50 % en poids, ou d'au moins 55 % en poids, ou d'au moins 60 % en poids, ou d'au moins 65 % en poids, ou d'au moins 70 % en poids, ou d'au moins 75 % en poids, ou d'au moins 80 % en poids, ou d'au moins 85 % en poids, ou d'au moins 90 % en poids, ou d'au moins 95 % en poids, dans chaque cas, par rapport au poids total de la composition cosmétique.

9. Composition cosmétique selon l'une quelconque des revendications précédentes, dans laquelle la teneur en poids du constituant tensioactif est de 1,0 % en poids, ou de 2,0 %, ou de 4,0 % en poids, ou d'au plus 6,0 % en poids, ou d'au plus 8,0 % en poids, ou d'au plus 10 % en poids, ou d'au plus 15 % en poids, ou d'au plus 20 % en poids, ou d'au plus 25 % en poids, ou d'au plus 30 % en poids, ou d'au plus 35 % en poids, ou d'au plus 40 % en poids, ou d'au plus 45 % en poids, ou d'au plus 50 % en poids, ou d'au plus 55 % en poids, ou d'au plus 60 % en poids, ou d'au plus 65 % en poids, ou d'au plus 70 % en poids, ou d'au plus 75 % en poids, ou d'au plus 80 % en poids, ou d'au plus 85 % en poids, ou d'au plus 90 % en poids, ou d'au plus 95 % en poids, dans chaque cas, par rapport au poids total de la composition cosmétique.

10. Composition cosmétique selon l'une quelconque des revendications précédentes, dans laquelle le constituant tensioactif comprend ou est essentiellement constitué d'un tensioactif choisi dans le groupe consistant de :
- des tensioactifs anioniques qui sont de préférence choisis dans : le laureth sulfate de sodium, le dodécylsulfate de sodium, le laurylsulfate de sodium, le laurylsulfate d'ammonium, les sulfosuccinates (par exemple le dioctyl sulfosuccinate, le laureth sulfosuccinate de disodium, le PEG-5 laurylcitrate sulfosuccinate disodique, le diéthylhexyl sulfosuccinate de sodium, le disodium ricinoléamido-MEA-sulfosuccinate, l'undécylénamido disodique MEA-sulfosuccinate), les taurates d'acyle-méthyle (par exemple le N-méthyl lauroyl taurate de sodium), les sulfonates d'alkylbenzène (par exemple le dodécylbenzène sulfonate), les sarcosinates d'acyle (par exemple le sarcosinate de lauroylsodium), les sulfonates glycérol éther gras (par exemple le (3-lauryloxy)-2-hydroxypropane sulfonate de sodium), l'acyliséthionate (par exemple le lauroyl iséthionate de sodium), les monoglycérides sulfates (par exemple le (3-lauroyloxy)-2-hydroxypropyle sulfate de sodium), les condensats de propylpeptides ;
- tensioactifs cationiques qui sont de préférence choisis dans : les chlorures de stéaralkonium (chlorure de benzyldiméthyloctadécylammonium), le chlorure de dicétyldimonium, le chlorure de docosyltriméthylammonium ;
- tensioactifs amphotères qui sont de préférence choisis dans : le lauraminopropionate de sodium, le cocoamphopropionate de sodium, les cocamidopropyl bétaïnes (par exemple lauramidopropyl bétaïne), la capryl/capramidopropyl bétaïne, l'undécylénamidopropyl bétaïne, le cocoamphodiacétate disodique, le cocoamphoacétate de sodium ;
- tensioactifs non ioniques qui sont de préférence choisis dans : le lauramide, le lauramide diéthanolamine, l'oxyde de lauramide, l'oxyde de stéaramine, les poloxamères (par exemple Poloxamer 101, Poloxamer 124), le PEG-7 cocoate de glycéryle, le laurate de glycéryle, la cocamide diéthanolamine, la cocamide monoéthanolamine ;
- bio-tensioactifs qui sont de préférence choisis dans : (i) les glycolipides (glucides-lipides) tels que les rhamnolipides, les lipides mannosyl-érythritol, les sophorolipides, les cellobioselipides et similaires ; (ii) les phospholipides ; (iii) les lipides de polyol ; (iv) les lipoprotéines ; (v) les lipopeptides ; (vi) les lipides d'ornithine ; (vii) les lipides neutres ; (viii) les lipides d'acides aminés ; (ix) les exolipides ; (x) les liposanes ; (xi) les sidérolipides ; (xii) les polyamines protéiques diglycosyl diglycérides ; (xiii) les fimbriae ; et les combinaisons de ceux-ci.

11. Composition cosmétique selon l'une quelconque des revendications précédentes, dans laquelle :
- le constituant cellodextrine comprend ou est essentiellement constitué de cellobiose ;
- la composition cosmétique comprend des particules solides comprenant ou constituées essentiellement du constituant cellodextrine sous forme non dissoute ;
- les particules ont une granulométrie moyenne, de préférence exprimée en valeur D50(v) déterminée par analyse par diffraction laser selon la norme ISO 13 320, d'au plus 100 µm, ou d'au plus 50 µm, ou d'au plus 10 µm, ou d'au plus 5,0 µm, ou d'au plus 1,0 µm, ou d'au plus 0,5 µm, ou d'au plus 0,1 µm, ou d'au plus 0,05 µm, ou d'au plus 0,01 µm, ou d'au plus 0,005 µm, ou d'au plus 0,001 µm ;
- la teneur en poids du constituant tensioactif est d'au moins 0,1 % en poids, de préférence d'au moins 1,0 % en poids, plus préférentiellement d'au moins 5,0 % en poids, encore plus préférentiellement d'au moins 10 % en poids, par rapport au poids total de la composition cosmétique ;
- la composition cosmétique comprend en outre de l'eau, dans laquelle la teneur en eau est d'au moins 20 % en poids, de préférence d'au moins 40 % en poids, plus préférentiellement d'au moins 60 % en poids, encore plus préférentiellement d'au moins 80 % en poids, par rapport au poids total de la composition cosmétique ;
- la composition cosmétique est choisie dans le savon liquide, le savon mousse, le gel douche et le shampooing capillaire.

12. Composition cosmétique selon l'une quelconque des revendications précédentes, dans laquelle la teneur en poids de cellobiose est d'au moins 1,0 % en poids, ou d'au moins 2,0 % en poids, ou d'au moins 3,0 % en poids, ou d'au moins 4,0 % en poids, ou d'au moins 5,0 % en poids, ou d'au moins 7,5 % en poids, ou d'au moins 10 % en poids, ou d'au moins 12,5 % en poids, ou d'au moins 15 % en poids, ou d'au moins 17,5 % en poids, ou d'au moins 20 % en poids, dans chaque cas, par rapport au poids total de la composition cosmétique.

13. Composition cosmétique selon l'une quelconque des revendications précédentes, qui comprend en outre un antioxydant.

14. Composition cosmétique selon l'une quelconque des revendications précédentes, qui présente une opacité d'au moins 25 %, ou d'au moins 30 %, ou d'au moins 35 %, ou d'au moins 40 %, ou d'au moins 45 %, ou d'au moins 50 %, ou d'au moins 55 %, ou d'au moins 60 %, ou d'au moins 65 %, ou d'au moins 70 %, ou d'au moins 75 %, ou d'au moins 80 %, ou d'au moins 85 %, ou d'au moins 90 %, ou d'au moins 95 %, ou d'au moins 100 %, déterminée par des mesures colorimétriques.

15. Composition cosmétique selon l'une quelconque des revendications précédentes, qui présente une intensité de reflets, sur une échelle de 0 à 10, d'au moins 1, ou d'au moins 2, ou d'au moins 3, ou d'au moins 4, ou d'au moins 5, ou d'au moins 6, ou d'au moins 7, ou d'au moins 8, ou d'au moins 9, déterminée au cours d'un test sensoriel selon la norme ISO 13 299.
